(19) Europäisches Patentamt
European Patent Office
Office européen des brevets

(11) Veröffentlichungsnummer : **0 462 931 B1**

# (12) EUROPÄISCHE PATENTSCHRIFT

(45) Veröffentlichungstag der Patentschrift :
**13.04.94 Patentblatt 94/15**

(51) Int. Cl.$^5$ : **C07D 249/18,** A01N 43/647

(21) Anmeldenummer : **91810449.8**

(22) Anmeldetag : **12.06.91**

(54) **Benzotriazol-1-sulfonylderivate mit mikrobiziden Eigenschaften.**

Die Akte enthält technische Angaben, die nach dem Eingang der Anmeldung eingereicht wurden und die nicht in dieser Patentschrift enthalten sind.

(30) Priorität : **21.06.90 CH 2065/90**

(43) Veröffentlichungstag der Anmeldung :
**27.12.91 Patentblatt 91/52**

(45) Bekanntmachung des Hinweises auf die Patenterteilung :
**13.04.94 Patentblatt 94/15**

(84) Benannte Vertragsstaaten :
**AT BE CH DE ES FR GB IT LI LU NL**

(56) Entgegenhaltungen :
**EP-A- 0 152 360**
**EP-A- 0 238 824**
**EP-A- 0 355 049**
**EP-A- 0 367 242**
**BE-A- 566 599**

(73) Patentinhaber : **CIBA-GEIGY AG**
**Klybeckstrasse 141**
**CH-4002 Basel (CH)**

(72) Erfinder : **Ackermann, Peter, Dr.**
**Hangelimattweg 113**
**CH-4148 Pfeffingen (CH)**
Erfinder : **Schellenbaum, Max, Dr.**
**Lutzerstrasse 14**
**CH-4132 Muttenz (CH)**

**EP 0 462 931 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue Benztriazolsulfonsäure-Derivate der nachstehenden Formel I. Sie betrifft ferner die Herstellung dieser Substanzen sowie agrochemische Mittel, die als Wirkstoff mindestens eine dieser Verbindungen enthalten. Die Erfindung betrifft ebenso die Herstellung der genannten Mittel sowie die Verwendung der Wirkstoffe oder der Mittel zur Bekämpfung oder Verhütung eines Befalls von Pflanzen durch phytopathogene Mikroorganismen, vorzugsweise Fungi.

Die erfindungsgemässen Verbindungen entsprechen der allgemeinen Formel I

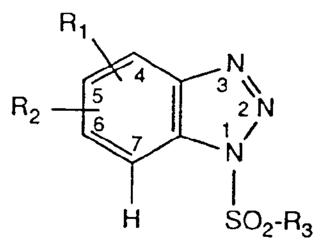

(I)

in welcher bedeuten:

$R_1$ Wasserstoff, Halogen, $C_1$-$C_4$-Alkyl, $C_1$-$C_4$-Alkoxy, $C_1$-$C_4$-Haloalkoxy mit mindestens zwei gleichen oder verschiedenen Halogenatomen, ferner $CF_3$, Nitro oder die Gruppe N(R')R", wobei R' und R" unabhängig voneinander $C_1$-$C_4$-Alkyl sind;

$R_2$ Phenoxy oder Phenylthio, welche unsubstituiert oder 1- bis 3-fach substituiert sind durch Halogen, $C_1$-$C_4$-Alkyl, $C_1$-$C_4$-Alkoxy, $C_1$-$C_4$-Haloalkoxy mit mindestens zwei gleichen oder verschiedenen Halogenatomen, ferner substituiert durch Cyano, Nitro, $C_2$-$C_4$-Alkenyl, $C_2$-$C_4$-Alkinyl oder in 3,4-Stellung durch einen Rest O-$CH_2$-O oder ferner substituiert durch Phenyl, 2-Phenylethinyl oder einen weiteren Phenoxyrest, der unsubstituiert oder durch Halogen, $C_1$-$C_4$-Alkyl und/oder $C_1$-$C_4$-Alkoxy substituiert ist;

$R_3$ Alkyl, Aryl oder Aralkyl mit maximal 14 C-Atomen, wobei diese Reste durch Halogen, $C_1$-$C_4$-Alkyl und/oder Nitro substituiert sind;

unter Einschluss der Stellungsisomeren von $R_1$ und $R_2$, sofern diese Substituenten die Positionen 5 oder 6 besetzen.

Unter dem Begriff Alkyl selbst oder als Bestandteil eines anderen Substituenten wie Alkoxy oder Haloalkoxy etc., sind je nach Zahl der angegebenen Kohlenstoffatome beispielsweise die folgenden geradkettigen oder verzweigten Gruppen zu verstehen: Methyl, Ethyl, Propyl, Butyl sowie ihre Isomeren, wie z.B. Isopropyl, Isobutyl, tert.-Butyl usw. Halogen und Halo stehen für Fluor, Chlor, Brom oder Jod. Haloalkoxy steht somit für einen einfach bis perhalogenierten Alkoxyrest, wie z.B. $OCH_2F$, $OCHF_2$, $OCHFCH_3$, $OCH_2CH_2Br$, $OCF_2CHFCl$ usw. $C_2$-$C_4$-Alkenyl steht für einen ungesättigten, aliphatischen Rest mit einer oder mit mehreren Doppelbindungen, so z.B. für Propen- 1 -yl, Allyl, Buten-1-yl, Buten-2-yl, Buten-3-yl, usw. Unter Alkinyl sind ungesättigte, aliphatische Reste und maximal 4 Kohlenstoffatome zu verstehen, z.B. Propargyl, Butin-2-yl, Butin-3-yl usw. Aryl und Aralkyl steht für z.B. Phenyl, Benzyl, Phenethyl, Naphthyl usw.

Die Verbindungen der Formel I sind bei Raumtemperatur stabile Oele oder Feststoffe, die sich durch wertvolle mikrobizide Eigenschaften auszeichnen. Sie lassen sich auf dem Agrarsektor oder verwandten Gebieten präventiv und kurariv zur Bekämpfung von pflanzenschädigenden Mikroorganismen einsetzen. Die erfindungsgemässen Wirkstoffe der Formel I zeichnen sich beiniedrigen Anwendungskonzentrationen nicht nur durch hervorragende mikrobizide, insbesondere fungizide, Wirkung sondern auch durch besonders gute Pflanzenverträglichkeit aus.

Folgende Wirkstoffgruppen sind aufgrund ihrer ausgeprägten pflanzenschützenden Eigenschaften bevorzugt:

Verbindungen der Formel I, worin bedeuten:

1a) $R_1$ Wasserstoff, Halogen, $C_1$-$C_4$-Alkyl, Methoxy, Ethoxy, $CF_3$, $NO_2$, $C_1$-$C_2$-Haloalkoxy mit mindestens wei Fluoratomen, oder ferner Diethylamin oder Dimethylamin;
und $R_2$ und $R_3$ die unter Formel I angegebenen Bedeutungen besitzen.

1b) $R_1$ Wasserstoff, Fluor, Chlor, Brom, Methyl, Ethyl, Methoxy, $CF_3$, $NO_2$, $C_1$-$C_2$-Haloalkoxy mit mindestens 2 Fluoratomen, oder ferner Dimethylamin;
und $R_2$ und $R_3$ die unter Formel I angegebenen Bedeutungen besitzen.

1c) $R_1$ Wasserstoff, Chlor, Brom, Methyl, Methoxy, $CF_3$, $NO_2$, Trifluormethoxy, Trifluorchlorethoxy,

Difluormethoxy oder Dimethylamin;
und $R_2$ und $R_3$ die unter Formel I angegebenen Bedeutungen besitzen.
1d) $R_1$ Chlor, Methyl, Methoxy, $CF_3$ oder Dimethylamin;
und $R_2$ und $R_3$ die unter Formel I angegebenen Bedeutungen besitzen.
2a) $R_2$ Phenoxy oder Phenylthio, welche unsubstituiert oder 1- oder 2-fach substituiert sind durch Fluor, Chlor oder Brom, $C_1$-$C_4$-Alkyl, $C_1$-$C_4$-Alkoxy, $C_1$-$C_2$-Haloalkoxy mit mindestens 2 Fluoratomen, ferner substituiert durch Nitro, Cyano, Allyl, Propargyl, 2-Phenylethinyl oder einen weiteren Phenoxyrest, der unsubstituiert oder durch Fluor, Chlor, Brom oder Methyl subsrituiert ist;
und $R_1$ und $R_3$ die unter Formel I angegebenen Bedeutungen besitzen.
2b) $R_2$ Phenoxy, das unsubstituiert oder subsrituiert ist durch Fluor, Chlor, Brom, $C_1$-$C_4$-Alkyl, $C_1$-$C_4$-Alkoxy, $C_1$-$C_2$-Haloalkoxy mit mindestens 2 Fluoratomen, ferner substituiert durch Cyano, Nitro, Allyl, Propargyl, 2-Phenylethinyl oder einen weiteren Phenoxyrest, der unsubstituiert oder durch Fluor, Chlor, Brom oder Methyl substituiert ist;
und $R_1$ und $R_3$ die unter Formel I angegebenen Bedeutungen besitzen.
2c) $R_2$ Phenylthio, das unsubstituiert oder substituiert ist durch Fluor, Chlor, $C_1$-$C_2$-Alkyl, $C_1$-$C_2$-Alkoxy, $C_1$-$C_2$-Haloalkoxy mit mindestens 2 Fluoratomen, ferner substituiert durch Nitro, Allyl, Propargyl oder 2-Phenylethinyl;
und $R_1$ und $R_3$ die unter Formel I angegebenen Bedeutungen besitzen.
2d) $R_2$ Phenoxy, das unsubstituiert oder substituiert ist durch Chlor, Brom, Methyl, tert.-Butyl, Methoxy, Ethoxy, Trifluormethoxy, Cyano, Nitro, Allyl, Propargyl, 2-Phenylethinyl oder einen weiteren Phenoxyrest, der unsubstituiert oder durch Fluor, Brom oder Methyl substituiert ist;
und $R_1$ und $R_3$ die unter Formel I angegebenen Bedeutungen besitzen.
3a) $R_3$ $C_1$-$C_4$-Alkyl, durch Halogen substituiertes $C_1$-$C_4$-Alkyl, Phenyl, durch Halogen, $C_1$-$C_4$-Alkyl und/oder Nitro substituiertes Phenyl, Benzyl oder durch Halogen, $C_1$-$C_4$-Alkyl und/oder Nitro substituiertes Benzyl;
und $R_1$ und $R_2$ die unter Formel I angegebenen Bedeutungen besitzen.
3b) $R_3$ $C_1$-$C_4$-Alkyl, durch Fluor oder Chlor substituiertes Alkyl, Phenyl, durch Chlor, Methyl oder Nitro substituiertes Phenyl, Benzyl oder durch Chlor, Methyl oder Nitro substituiertes Benzyl;
und $R_1$ und $R_2$ die unter Formel I angegebenen Bedeutungen besitzen.
4a) $R_1$ Wasserstoff, Halogen, $C_1$-$C_4$-Alkyl, Methoxy, Ethoxy, $CF_3$, $NO_2$, $C_1$-$C_2$-Haloalkoxy mit mindestens 2 Fluor- und/oder 2 Chloratomen, oder ferner Diethylamin oder Dimethylamin;
$R_2$ Phenoxy oder Phenylthio, welche unsubstituiert oder 1- oder 2-fach substituiert sind durch Fluor, Chlor oder Brom, $C_1$-$C_4$-Alkyl, $C_1$-$C_4$-Alkoxy, $C_1$-$C_2$-Haloalkoxy mit mindestens 2 Fluoratomen, ferner substituiert durch Cyano, Nitro, Allyl, Propargyl, 2-Phenylethinyl oder einen weiteren Phenoxyrest, der unsubstituiert oder durch Fluor, Chlor, Brom oder Methyl substituiert ist;
$R_3$ $C_1$-$C_4$-Alkyl, durch Halogen substituiertes $C_1$-$C_4$-Alkyl, Phenyl, durch Halogen, $C_1$-$C_4$-Alkyl und/oder Nitro substituiertes Phenyl, Benzyl oder durch Halogen, $C_1$-$C_4$-Alkyl und/oder Nitro substituiertes Benzyl.
4b) $R_1$ Wasserstoff, Fluor, Chlor, Brom, Methyl, Ethyl, Methoxy, $CF_3$, $NO_2$, $C_1$-$C_2$-Haloalkoxy mit mindestens 2 Fluoratomen, oder ferner Dimethylamin;
$R_2$ Phenoxy, das unsubsrituiert oder subsrituiert ist durch Fluor, Chlor, Brom, $C_1$-$C_4$-Alkyl, $C_1$-$C_4$-Alkoxy, $C_1$-$C_2$-Haloalkoxy mit mindestens 2 Fluoratomen, ferner substituiert durch Cyano, Niao, Allyl, Propargyl, 2-Phenylethinyl oder einen weiteren Phenoxyrest, der unsubstituiert oder durch Fluor, Brom oder Methyl substituiert ist;
$R_3$ $C_1$-$C_4$-Alkyl, durch Fluor oder Chlor substituiertes Alkyl, Phenyl, durch Chlor, Methyl oder Nitro substituiertes Phenyl, Benzyl oder durch Chlor, Methyl oder Nitro substituiertes Benzyl.
4c) $R_1$ Wasserstoff, Chlor, Brom, Methyl, Methoxy, $CF_3$, $NO_2$, Trifluormethoxy, Trifluorchlorethoxy, Difluormethoxy oder Dimethylamin;
$R_2$ Phenylthio, das unsubstituiert oder subsrituiert ist durch Fluor, Chlor, $C_1$-$C_2$-Alkyl, $C_1$-$C_2$-Alkoxy, $C_1$-$C_2$-Haloalkoxy mit mindestens 2 Fluoratomen, ferner substituiert durch Nitro, Allyl, Propargyl oder 2-Phenylethinyl;
$R_3$ $C_1$-$C_4$-Alkyl, durch Fluor oder Chlor substituiertes Alkyl, Phenyl, durch Chlor, Methyl oder Nitro substituiertes Phenyl, Benzyl oder durch Chlor, Methyl oder Nitro substituiertes Benzyl;
und $R_1$ und $R_2$ die unter Formel I angegebenen Bedeutungen besitzen.
4d) $R_1$ Chlor, Methyl, Methoxy, $CF_3$ oder Dimethylamin;
$R_2$ Phenoxy, das unsubsrituiert oder substituiert ist durch Chlor, Brom, Methyl, tert.-Butyl, Methoxy, Ethoxy, Trifluormethoxy, Cyano, Nitro, Allyl, Propargyl, 2-Phenylethinyl oder einen weiteren Phenoxyrest, der unsubstituiert oder durch Fluor, Brom oder Methyl substituiert ist;
$R_3$ $C_1$-$C_4$-Alkyl, durch Fluor oder Chlor substituiertes Alkyl, Phenyl, durch Chlor, Methyl oder Nitro substituiertes Phenyl, Benzyl oder durch Chlor, Methyl oder Nitro substituiertes Benzyl;

In ihrer Wirkungsweise bevorzugte Verbindungen der Formel I sind folgende:
1-N-Methansulfonyl-5-chlor-6-phenoxy-benztriazol;
1-N-Methansulfonyl-5-chlor-6-(2-chlorphenoxy)-benztriazol;
1-N-Methansulfonyl-5-chlor-6-(4-chlorphenoxy)-benztriazol;
1-N-Methansulfonyl-5-chlor-6-(2,4-dibromphenoxy)-benztriazol;
1-N-Methansulfonyl-5-chlor-6-(2-fluorphenoxy)-benztriazol;
1-N-Methansulfonyl-5-chlor-6-(4-ethoxyphenoxy)-benztriazol;
3-N-Methansulfonyl-5-chlor-6-phenoxy-benztriazol;
3-N-Methansulfonyl-5-chlor-6-(2-chlorphenoxy)-benztriazol;
3-N-Methansulfonyl-5-chlor-6-(4-chlorphenoxy)-benztriazol;
3-N-Methansulfonyl-5-chlor-6-(2,4-dibromphenoxy)-benztriazol;
3-N-Methansulfonyl-5-chlor-6-(2-fluorphenoxy)-benztriazol; und
3-N-Methansulfonyl-5-chlor-6-(4-ethoxyphenoxy)-benztriazol;
sowie die Gemische der 1-N- und 3-N-substituierten Strukturisomeren.

Die Verbindungen der Formel I werden hergestellt

1) durch Umsetzung einer Verbindung der Formel II

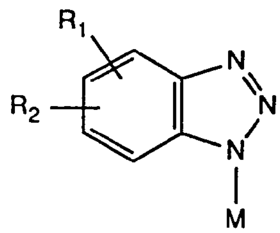

(II)

mit einer Verbindung der Formel III

$$Q\text{-}SO_2\text{-}R_3 \quad \text{(III)}$$

worin $R_1$, $R_2$ und $R_3$ die unter Formel I angegebenen Bedeutungen besitzen und M Wasserstoff oder ein Alkalimetall, bevorzugt Na, K oder Li, und Q ein Halogenatom, bevorzugt Chlor, oder den Rest $O\text{-}SO_2\text{-}R_3$ darstellen, in einem inerten Lösungsmittel, gegebenenfalls in Gegenwart einer Base, bei Temperaturen von -30° bis 180°C, bevorzugt -10° bis 80°C, unter Normaldruck, vermindertem oder erhöhtem Druck, bevorzugt Normaldruck; oder

2) durch Diazotierung einer Verbindung der Formel IV

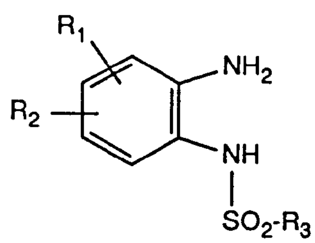

(IV)

worin $R_1$, $R_2$ und $R_3$ die unter Formel I angegebenen Bedeutungen besitzen, entweder

a) mit einem anorganischen Nitrit in einem Lösungsmittel, bevorzugt in Wasser oder in Gemischen von Wasser mit Alkoholen, bevorzugt Methanol oder Ethanol, oder in Gemischen von Wasser mit Ether, bevorzugt Dioxan oder Dimethoxyethan, in Gegenwart einer Säure bei Temperaturen von -30° bis 180°C, bevorzugt 0° bis 80°C; oder

b) mit einem organischen Nitrit, bevorzugt Ethylnitrit, Amylnitrit oder tert.Butylnitrit, in einem Lösungsmittel, bevorzugt Alkohole wie Methanol oder Ethanol oder Ether wie Dioxan oder Dimethoxyethan, gegebenenfalls in Gegenwart einer Säure, bei Temperaturen von -30° bis 180°C, bevorzugt 0° bis 80°C, unter Normaldruck oder erhöhtem Druck.

Verbindungen der Formel IIa

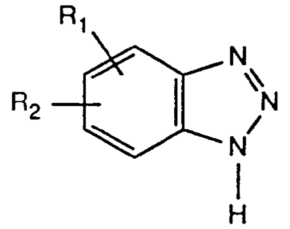

(IIa),

worin bedeuten:

$R_1$ Wasserstoff, Halogen, $C_1$-$C_4$-Alkyl, $C_1$-$C_4$-Alkoxy, $C_1$-$C_4$-Haloalkoxy mit mindestens zwei gleichen oder verschiedenen Halogenatomen, ferner $CF_3$, Nitro oder die Gruppe N(R')R", wobei R' und R" unabhängig voneinander $C_1$-$C_4$-Alkyl sind;

$R_2$ Phenoxy oder Phenylthio, welche unsubstituiert oder 1- bis 3-fach substituiert sind durch Halogen, $C_1$-$C_4$-Alkyl, $C_1$-$C_4$-Alkoxy, $C_1$-$C_4$-Haloalkoxy mit mindestens zwei gleichen oder verschiedenen Halogenatomen, ferner substituiert durch Cyano, Nitro, $C_2$-$C_4$-Alkenyl, $C_2$-$C_4$-Alkinyl, Phenyl, 2-Phenylethinyl oder einen weiteren Phenoxyrest, der unsubstituiert oder durch Halogen, $C_1$-$C_4$-Alkyl und/oder $C_1$-$C_4$-Alkoxy substituiert ist;

$R_3$ Alkyl, Aryl oder Aralkyl mit maximal 14 C-Atomen, wobei diese Reste durch Halogen, $C_1$-$C_4$-Alkyl und/oder Nitro substituiert sind;

sind neu mit Ausnahme der Verbindungen, in denen gleichzeitig $R_1$ Wasserstoff und $R_2$ 5-(2-Chlor-4-trifluormethylphenoxy) und $R_1$ 6-Chlor und $R_2$ 5-Phenoxy darstellen.

Die Verbindungen der Formel IIa werden hergestellt

3) durch Diazotierung einer Verbindung der Formel V

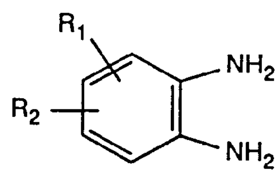

(V)

worin $R_1$ und $R_2$ die unter Formel IIa angegebenen Bedeutungen besitzen, entweder

3a) mit einem anorganischen Nitrit in einem Lösungsmittel, bevorzugt in Wasser oder in Gemischen von Wasser mit Alkoholen, bevorzugt Methanol oder Ethanol, oder in Gemischen von Wasser mit Ether, bevorzugt Dioxan oder Dimethoxyethan, in Gegenwart einer Säure bei Temperaturen von -30° bis 180°C, bevorzugt 0° bis 80°C; oder

3b) mit einem organischen Nitrit, bevorzugt Ethylnitrit, Amylnitrit oder tert.Butylnitrit, in einem Lösungsmittel, bevorzugt Alkohole wie Methanol oder Ethanol oder Ether wie Dioxan oder Dimethoxyethan, gegebenenfalls in Gegenwart einer Säure bei Temperaturen von -30° bis 180°C, bevorzugt 0° bis 80°C, unter Normaldruck oder erhöhtem Druck; oder

4) durch Umsetzung einer Verbindung der Formel VI

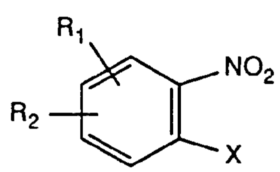

(VI),

worin $R_1$ und $R_2$ die unter Formel IIa angegebenen Bedeutungen besitzen und X ein Halogenatom, bevorzugt Fluor oder Chlor, darstellt mit Hydrazin oder Hydrazinhydrat in einem Lösungsmittel, bevorzugt Alkohole wie Methanol oder Ethanol, in Gegenwart eines säurebindenden Mittels, bevorzugt Natriumcarbonat, Kaliumcarbonat oder Triethylamin, bei Rückflusstemperaturen des Reaktionsmediums zu einer Verbindung der Formel VII

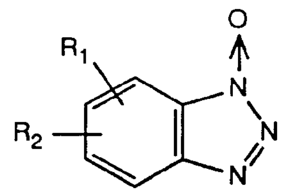

(VII),

welche anschliessend mit einem Chlorketon der Formel VIII

$$Cl\text{-}CH_2\text{-}CO\text{-}(C_1\text{-}C_6\text{- Alkyl}) \quad (VIII),$$

vorzugsweise Chloraceton, in einem inerten Lösungsmittel in Gegenwart einer Base bei Temperaturen von 40° bis 140°C, vorzugsweise 60° bis 120°C, zur Reaktion gebracht wird.

In den vorstehend beschriebenen Verfahren kommen folgende inerte Lösungsmittel in Frage: aliphatische, cycloaliphatische oder aromatische Kohlenwasserstoffe, wie z.B. Hexan, Cyclohexan, Toluol, Xylol, Petrolether oder Ligroin; chlorierte Kohlenwasserstoffe, wie z.B. Methylenchlorid, Chloroform, Tetrachlorkohlenstoff, Chlorbenzol; Ether, wie z.B. Diethylether, Diisopropylether, Furan, Tetrahydrofuran, Dioxan; Ketone, wie z.B. Aceton, Methylethylketon; Alkohole, wie z.B. Methanol, Ethanol, Isopropanol; Ester, wie z.B. Essigsäureethylester, Essigsäurebutylester; Nitrile wie z.B. Acetonitril, Propionitril; Säureamide, wie z.B. Dimethylformamid; Sulfone und Sulfoxid, wie z.B. Dimethylsulfoxid, Sulfolan.

Als Basen oder säurebindende Mittel eignen sich z.B. Hydroxide, Carbonate, Hydrogencarbonate oder Alkoholate der Alkalimetalle; sowie tertiäre Amine, wie z.B. Triethylamin, Triisopropylamin, Pyridin oder 4-N,N-Dimethylaminopyridin.

Die vorstehend beschriebenen Verfahren entsprechen Synthesemethoden, die aus der Literatur bekannt sind. Sie sind z.B. beschrieben in Chem. Reviews 46, 1 (1950) und in der deutschen Offenlegungsschrift 34 06 011. Die Synthese von Ausgangsverbindungen des Typs der Formel IV ist aus der US-Patentschrift 2,943,017 bekannt.

Verbindungen mit Benztriazolsulfonsäure-Strukturen sind bereits bekannt. Derartige Substanzen sind in den Patentschriften BE-566599, DE-1046937, GB-885,843 und US-2,943,017 als fungizid verwendbare Wirkstoffe beschrieben. Benzuriazole sind als Herbizide in der EP-A-355049 und EP-A-367242 offenbart. Phenylsulfonylazole sind als Mikrobizide Mittel aus EP-A-238824 bekannt.

Die bekannten Wirkstoffe können jedoch die heutzutage in der praktischen Nutzanwen dung als Fungizide an sie gestellten Forderungen, vor allem beim Einsatz niedriger Aufwandmengen sowie gegen spezielle Schädlinge, nicht immer in befriedigendem Masse erfüllen.

Ueberraschenderweise wurde nun gefunden, dass Verbindungen der Formel I ein für praktische Bedürfnisse sehr günstiges biozides Spektrum zur Bekämpfung von phytopathogenen Mikroorganismen, insbesondere Fungi, aufweisen. Sie besitzen sehr vorteilhafte kurative, präventive und insbesondere systemische Eigenschaften und werden zum Schutz von zahlreichen Kulturpflanzen eingesetzt. Mit den Wirkstoffen der Formel I können an Pflanzen oder Pflanzenteilen (Früchte, Blüten, Laubwerk, Stengel, Knollen, Wurzeln) von unterschiedlichen Nutzkulturen die auftretenden Schädlinge eingedämmt oder vernichtet werden, wobei auch später zuwachsende Pflanzenteile z.B. vor phytopathogenen Mikroorganismen verschont bleiben.

Die neuen Wirkstoffe der Formel I erweisen sich als bevorzugt wirksam gegen spezielle Gattungen der Pilzklasse Fungi imperfecti (z.B. Cercospora), Basidiomyceten (z.B. Puccinia) sowie Ascomyceten (z.B. Erysiphe und Venturia) und insbesondere gegen Oomyceten (z.B. Plasmopara und Phytophthora). Sie stellen damit im Pflanzenschutz eine wertvolle Ergänzung der Mittel für die Bekämpfung von phytopathogenen Pilzen dar. Für ihre Anwendung in der Praxis besitzen sie vorteilhafterweise sowohl kurative und präventive als auch systemische Eigenschaften und können zum Schutz von zahlreichen Kulturpflanzen eingesetzt werden. Mit diesen Wirkstoffen werden an Pflanzen oder Pflanzenteilen (Früchte, Blüten, Laubwerk, Stengel, Knollen, Wurzeln) von unterschiedlichen Nutzkulturen die auftretenden Schädlinge eingedämmt oder vernichtet, wobei auch später zuwachsende Pflanzenteile z.B. vor phytopathogenen Pilzen verschont bleiben. Die Verbindungen der Formel I können ferner als Beizmittel zur Behandlung von Saatgut (Früchte, Knollen, Körner) und Pflanzenstecklingen zum Schutz vor Pilzinfektionen sowie gegen im Erdboden auftretende phytopathogene Pilze eingesetzt werden.

Die Erfindung betrifft auch die Mittel, die als Wirkstoffkomponente Verbindungen der Formel I enthalten, insbesondere pflanzenschützende Mittel, sowie deren Verwendung auf dem Agrarsektor oder verwandten Gebieten.

Darüber hinaus schliesst die vorliegende Erfindung auch die Herstellung dieser Mittel ein, die gekennzeichnet ist durch das innige Vermischen der Aktivsubstanz mit einem oder mehreren hierin beschriebenen Substanzen bzw. Substanzgruppen. Eingeschlossen ist auch ein Verfahren zur Behandlung von Pflanzen, das

sich durch Applikation der neuen Verbindungen der Formel I bzw. der neuen Mittel auszeichnet.

Als Zielkulturen für den hierin offenbarten pflanzenschützenden Einsatz gelten im Rahmen dieser Erfindung beispielsweise folgende Pflanzenarten: Getreide (Weizen, Gerste, Roggen, Hafer, Reis, Mais, Sorghum und verwandte Species); Rüben (Zucker- und Futterrüben); Kern-, Stein und Beerenobst (Aepfel, Birnen, Pflaumen, Pfirsiche, Mandeln, Kirschen, Erd-, Him- und Brombeeren); Hülsenfrüchte (Bohnen, Linsen, Erbsen, Soja); Oelkulturen (Raps, Senf, Mohn, Oliven, Sonnenblumen, Kokos, Rizinus, Kakao, Erdnüsse); Gurkengewächse (Kürbis, Gurken, Melonen); Fasergewächse (Baumwolle, Flachs, Hanf, Jute); Citrusfrüchte (Orangen, Zitronen, Pampelmusen, Mandarinen); Gemüsesorten (Spinat, Kopfsalat, Spargel, Kohlarten, Möhren, Zwiebeln, Tomaten, Kartoffeln, Paprika); Lorbeergewächse (Avocado, Cinnamonium, Kampfer) oder Pflanzen wie Tabak, Nüsse, Kaffee, Zuckerrohr, Tee, Pfeffer, Weinreben, Hopfen, Bananen- und Naturkautschukgewächse sowie Zierpflanzen.

Wirkstoffe der Formel I werden üblicherweise in Form von Zusammensetzungen verwendet und können gleichzeitig oder nacheinander mit weiteren Wirkstoffen auf die zu behandelnde Fläche oder Pflanze gegeben werden. Diese weiteren Wirkstoffe können sowohl Düngemittel, Spurenelemente-Vermittler oder andere das Pflanzenwachstum beeinflussende Präparate sein. Es können dabei auch selektive Herbizide sowie Insektizide, Fungizide, Bakterizide, Nematizide, Molluskizide oder Gemische mehrerer dieser Präparate zusammen mit gegebenenfalls weiteren in der Formulierungstechnik üblichen Trägerstoffen, Tensiden oder anderen applikationsfördernden Zusätzen Verwendung finden.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln.

Ein bevorzugtes Verfahren zum Aufbringen eines Wirkstoffs der Formel I bzw. eines agrochemischen Mittels, das mindestens einen dieser Wirkstoffe enthält, ist das Aufbringen auf das Blattwerk (Blattapplikation). Applikationsfrequenz und Aufwandmenge richten sich dabei nach dem Befallsdruck des betreffenden Erregers. Die Wirkstoffe der Formel I können aber auch über den Erdboden durch das Wurzelwerk in die Pflanze gelangen (systemische Wirkung), indem man den Standort der Pflanze mit einer flüssigen Zubereitung tränkt oder die Substanzen in fester Form in den Boden einbringt, z.B. in Form von Granulat (Bodenapplikation). Bei Wasserreiskulturen kann man solche Granulate dem überfluteten Reis-Feld zudosieren. Die Verbindungen der Formel I können aber auch auf Samenkörner aufgebracht werden (Coating), indem man die Körner entweder in einer flüssigen Zubereitung des Wirkstoffs tränkt oder sie mit einer festen Zubereitung beschichtet.

Die Verbindungen der Formel I werden dabei in unveränderter Form oder vorzugsweise zusammen mit den in der Formulierungstechnik üblichen Hilfsmitteln eingesetzt. Dafür werden sie zweckmässigerweise z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt. Günstige Aufwandmengen liegen im allgemeinen bei 10 g bis 5 kg Aktivsubstanz (AS) je ha, bevorzugt 20 g bis 1 kg AS/ha.

Die Formulierungen d.h. die den Wirkstoff der Formel I und gegebenenfalls einen festen oder flüssigen Zusatzstoff enthaltenden Mittel, Zubereitungen oder Zusammensetzungenwerden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel kommen in Frage: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder Ethylether, Ketone wie Cyclohexanon, stark polare Lösungsmittel, wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorilonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht-sorptive Trägermaterialien, z.B. Calcit oder Sand, in Frage. Darüber hinaus kann eine Vielzahl von vorgranulierten Materialien anorganischer Natur wie insbesondere Dolomit oder zerkleinerte Pflanzenrückstände verwendet werden.

Als oberflächenaktive Verbindungen kommen je nach Art des zu formulierenden Wirkstoffs der Formel I nichtionogene, kation-und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen, als auch wasserlösliche synthetische obeflächenaktive Verbindungen sein.

Als Seifen seien die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können. Ferner sind auch die Fettsäure-Methyllaurinsalze zu erwähnen.

Häufiger werden jedoch sog. synthetische Tenside verwendet, insbesondere Alkansulfonate, Fettalkoholsulfate, sulfonierte Benzimidazolderivate oder Alkylsulfonate.

Die Fettalkoholsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na-oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Ethylenoxyd-Addukten. Die sulfonierten Benzimidazolderivate enthalten vorzugsweise 2-Sulfonsäuregruppen und einen Fettsäurerest mit 8-22 C-Atomen. Alkylarylsulfonate sind z.B. die Na-, Ca- oder Triethanolaminsalze der Dodecylbenzolsulfonsäure, der Dibutylnaphthalinsulfonsäure oder eines Naphthalinsulfonsäure-Formaldehydkondensarionsproduktes.

Ferner kommen auch entsprechende Phosphate wie z.B. Salze des Phosphorsäureesters eines p-Nonylphenol-(4-14)-Ethylenoxyd-Adduktes in Frage.

Als nichtionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind wasserlösliche 20 bis 250 Aethylenglykoläthergruppen und 10 bis 100 Propylenglykolethergruppen enthaltende Polyethylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheiten 1 bis 5 Ethylenglykoleinheiten.

Als Beispiele nichtionischer Tenside seien Nonylphenolpolyethoxyethanole, Ricinusölpolyglykolether, Polypropylen-Polyethylenoxydaddukte, Tributylphenoxypolyethylenethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi(2-chlorethyl)-ammoniumbromid.

Weitere in der Formulierungstechnik gebräuchlichen Tenside sind dem Fachmann bekannt oder können der einschlägigen Fachliteratur entnommen werden.

Die agrochemischen Zubereitungen enthalten in der Regel 0,1 bis 99 %, insbesondere 0,1 bis 95 %, Wirkstoff der Formel I, 99,9 bis 1 %, insbesondere 99,9 bis 5 %, eines festen oder flüssigen Zusatzstoffes und 0 bis 25 %, insbesondere 0,1 bis 25 %, eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer,
Viskositätsregulatoren, Bindemittel, Haftmittel sowie Dünger oder andere Wirkstoffe zur Erzielung spezieller Effekte enthalten.

Die nachfolgenden Beispiele dienen zur näheren Erläuterung der Erfindung, ohne dieselbe einzuschränken.

1. Herstellungsbeispiele

1.1 Herstellung von 5(2,3-Dichlorphenoxy)-4-chloro-2-nitroanilin

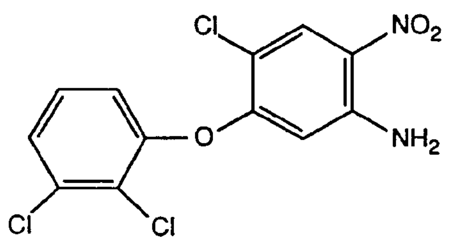

Ein Gemisch von 171 g 2,3-Dichlorphenol und 186 g 4,5-Dichlor-2-nitroanilin wird auf 150°C erwärmt. Zur dunkelroten Flüssigkeit tropft man innerhalb von 1 Stunde 101 g einer 50 %igen Kalilauge. Das gebildete Reaktionswasser wird mit wenig Phenol abdesrilliert. Man rührt über Nacht bei 150°C und gibt dann bei dieser Temperatur 450 ml Dimethylformamid zum Reaktionsgemisch. Die Lösung lässt man abkühlen und giesst alles auf ein Gemisch von 31 Wasser und 150 ml konz. Natronlauge. Die grünliche Suspension wird 1 Stunde bei Raumtemperatur gerührt, die gebildeten Kristalle werden abfiltriert und mit Wasser neutral gewaschen und dann getrocknet. Die noch feuchten gelblichen Kristalle werden roh in der nächsten Reaktionsstufe eingesetzt.

1.2 Herstellung von 4-Chlor-5-(2,3-dichlorphenoxy)-o-phenylendiamin

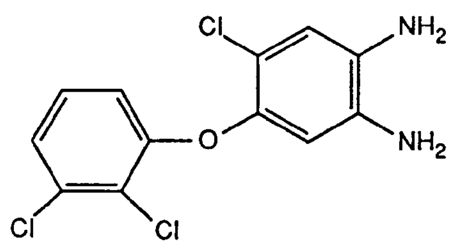

Zu einer Suspension von 80 g Eisenpulver in 400 ml Wasser tropft man innerhalb von 80 Minuten 17 ml Eisessig und erwärmt anschliessend auf Rückflusstemperatur. Nach der Zugabe von 100 ml Chlorbenzol versetzt man das Reaktionsgemisch innerhalb von 2 Stunden mit dem unter Beispiel 1.1 hergestellten Nitroanilin. Dabei wird eine exotherme Reaktion beobachtet. Nach 14-stündigem Erhitzen auf Rückfluss ist die Reduktion der Nitrogruppe beendet. Man lässt abkühlen, verdünnt mit 500 ml Chlorbenzol und stellt die wässrige Phase mit Soda alkalisch. Die organische Phase wird abgetrennt, mit Natriumsulfat getrocknet und unter vermindertem Druck eingeengt. Nach Zugabe von 100 ml Petroläther zum Rohprodukt erhält man eine kristalline Masse, welche abfiltriert und mit Petroläther gewaschen wird. Man erhält die Titelverbindung mit einem Schmelzpunkt von 110-112°C.

1.3 Herstellung von 5-Chlor-6-(2,3-dichlorphenoxy)-benztriazol

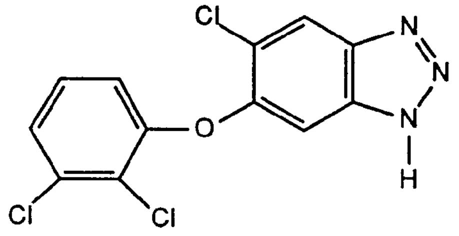

7,5 g des unter Beispiel 1.2 hergestellten Diamins werden in 50 ml Wasser suspendiert und bei Raumtemperatur mit 2,9 ml Essigsäure versetzt. Nach kurzem Erwärmen auf 60°C wird die Reaktionslösung auf 5°C abgekühlt und bei dieser Temperatur mit einer Lösung von 3,9 g Natriumnitrit in 12 ml Wasser versetzt. Man erwärmt nun während 3 Stunden auf 80°C, lässt die braune Suspension abkühlen und filtriert ab. Die erhaltenen Kristalle werden zweimal aus Aethanol/Wasser umskritallisiert und man erhält die Titelverbindung mit

einem Schmelzpunkt von 173°C.

1.4 Herstellung des Isomerengemisches 1-N-(Methansulfonyl)- resp. 3-N-(Methansulfonyl)-5-chlor-6-(2,3-dichlorphenoxy)-benztriazol

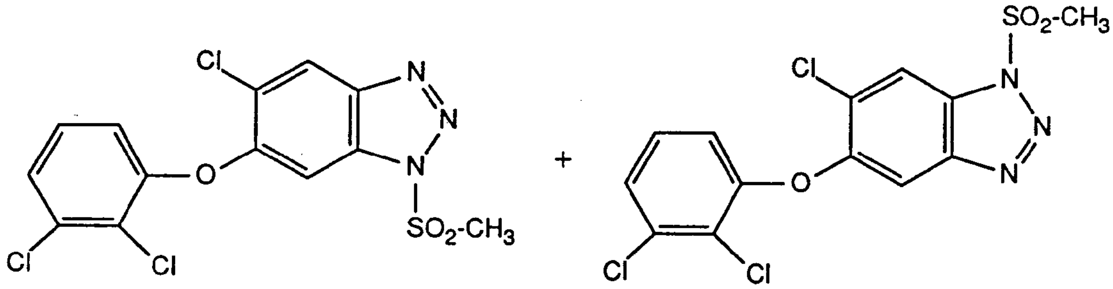

3,1 g Benztriazol aus Beispiel 1.3 werden in 30 ml Dimethylformamid und 2,78 ml Triäthylamin gelöst. Zur braunen Lösung tropft man ohne externe Heizung 1,56 ml Methansulfonylchlorid. Dabei steigt die Temperatur bis auf 45°C an. Bei Raumtemperatur lässt man anschliessend 1 Stunde weiterrühren und giesst die Suspension in 500 ml Wasser. Das ausfallende weisse Produkt wird abfiltriert und aus Aethanol umkristallisiert. Man erhält das gewünschte Produkt als Isomerengemisch mit einem Schmelzpunkt von 178-180°C.

Tabelle 1:

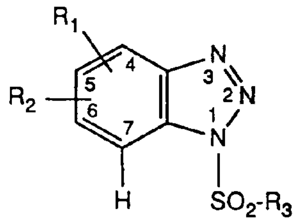

($R_1$, $R_2$: Position 5 oder 6)*

(Ph = Phenyl)

| Verb. Nr. | $R_1$ | $R_2$ | $R_3$ | Physikal. Daten |
|---|---|---|---|---|
| 1.1 | Cl | O-Ph | $CH(CH_3)_2$ | |
| 1.2 | $CH_3$ | O-Ph | $C_2H_5$ | |
| 1.3 | Cl | O-Ph | $CH_3$ | Smp. 100-104°C |
| 1.4 | $CH_3$ | O-Ph | $CH_3$ | |
| 1.5 | $CH_3$ | O-Ph | $CH(CH_3)_2$ | |
| 1.6 | Cl | O-Ph-2-Cl | $CH_3$ | Smp. 152-154°C |
| 1.7 | Cl | O-Ph-2-Cl | $C_2H_5$ | |
| 1.8 | Cl | O-Ph-2-Cl | $C_6H_5$ | |
| 1.9 | Cl | O-Ph-3-Cl | $CH_3$ | Smp. 76- 79°C |
| 1.10 | Cl | O-Ph-4-Cl | $C_3H_7$(n) | |
| 1.11 | Cl | O-Ph-4-Cl | $CH_2$-Ph | |
| 1.12 | Cl | O-Ph-4-Cl | $C_6H_5$ | |
| 1.13 | Cl | O-Ph-4-Cl | $CH_3$ | Smp. 144-145°C |
| 1.14 | Cl | O-Ph-2-F | $CH_3$ | Smp. 132-133°C |
| 1.15 | Cl | O-Ph-2-Br | $C_4H_9$ | |
| 1.16 | Cl | O-Ph-2,4-$(Br)_2$ | $CH_3$ | Smp. 147-148°C |
| 1.17 | $CH_3$ | O-Ph-4-Cl | $CH_3$ | |
| 1.18 | $OCF_3$ | O-Ph-2-Cl | $CH_3$ | |
| 1.19 | $NO_2$ | O-Ph | $C_6H_5$ | |
| 1.20 | $NO_2$ | O-Ph-4-Cl | $CH_3$ | |
| 1.21 | $C_4H_9$ | O-Ph-4-$C(CH_3)_3$ | $C_3H_7$(n) | |
| 1.22 | $C_4H_9$ | S-Ph | $CH_3$ | |
| 1.23 | $N(CH_3)_2$ | O-Ph-4-Cl | $CH_3$ | |
| 1.24 | $N(CH_3)_2$ | O-Ph-2-F | $CH_3$ | |

* Isomerengemische bezüglich $R_1$ und $R_2$ in Position 5 oder 6

| Verb. Nr. | $R_1$ | $R_2$ | $R_3$ | Physikal. Daten |
|---|---|---|---|---|
| 1.25 | $N(CH_3)_2$ | S-Ph-4-Cl | $CH_3$ | |
| 1.27 | $OCF_2CFHCF_3$ | O-Ph-4-Cl | $CH_3$ | |
| 1.28 | O-$CF_2CFClH$ | S-Ph-4-Cl | $CH_3$ | |
| 1.29 | O-$CF_2CFClH$ | S-Ph-4-Cl | $CH(CH_3)_2$ | |
| 1.30 | Cl | O-Ph-4-$OC_2H_5$ | $CH_3$ | Smp. 164-166°C |
| 1.31 | Cl | O-Ph-4-O-Ph | $CH_3$ | Smp. 161-163°C |
| 1.32 | Cl | O-Ph-4-(O-Ph-4-F) | $CH_3$ | |
| 1.33 | Cl | O-Ph-4-Ph | $CH_3$ | Smp. 200-205°C |
| 1.34 | Cl | O-Ph-4-Ph | $CH(CH_3)_3$ | |
| 1.35 | Cl | O-Ph-4-(O-Ph-4-$CH_3$) | $CH_3$ | |
| 1.36 | Cl | O-Ph-4-(C≡C-Ph) | $CH_3$ | |
| 1.37 | Cl | O-Ph-4-Allyl | $CH_3$ | |
| 1.38 | Cl | O-Ph-3,5-$(Cl)_2$ | $CH_3$ | Smp. 143-145°C |
| 1.39 | Cl | O-Ph-2,3-$(CH_3)_2$ | $CH_3$ | Smp. 140-144°C |
| 1.40 | Cl | O-Ph-2,3-$(Cl)_2$ | $CH(CH_3)_2$ | Smp. 95- 98°C |
| 1.41 | Cl | O-Ph-2,3-$(Cl)_2$ | $C_3H_7$(n) | Smp. 94- 96°C |
| 1.42 | Cl | O-Ph-2,3-$(Cl)_2$ | $C_6H_5$ | Smp. 115-118°C |
| 1.43 | Cl | O-Ph-2,3-$(Cl)_2$ | Ph-4-$CH_3$ | Smp. 134-137°C |
| 1.44 | Cl | O-Ph-2,3-$(Cl)_2$ | Ph-4-$NO_2$ | Smp. 172-175°C |
| 1.45 | Cl | O-Ph-2,3-$(Cl)_2$ | Ph-4-Cl | Smp. 130-132°C |
| 1.46 | Cl | S-Ph-4-Cl | $CH_3$ | Smp. 135-138°C |
| 1.47 | Cl | S-Ph-4-$CH_3$ | $CH_3$ | |
| 1.48 | Cl | O-Ph-2,4-$(F)_2$ | $CH_3$ | |
| 1.49 | Cl | O-Ph-2-Cl-4-F | $CH_3$ | Smp. 191-193°C |
| 1.50 | Cl | O-Ph-3-$NO_2$ | $CH_3$ | |
| 1.51 | Cl | O-Ph-2-$NO_2$-4-Cl | $CH_3$ | |
| 1.52 | Cl | O-Ph-4-$OCF_3$ | $CH_3$ | |
| 1.53 | $CH_3$ | O-Ph-3-$OC_2H_5$ | $CH_3$ | |
| 1.54 | $CH_3$ | O-Ph-4-CN | $CH_3$ | |
| 1.55 | Cl | O-Ph-2,3-$(Cl)_2$ | $CH_2$-Ph | |
| 1.56 | Cl | O-Ph-2,3-$(Cl)_2$ | $CH_2CF_3$ | |
| 1.57 | Cl | O-Ph-2,3-$(Cl)_2$ | $CH_3$ | Smp. 178-180°C |
| 1.58 | Cl | O-Ph-2,3-$(Cl)_2$ | $C_2H_5$ | |
| 1.59 | Cl | O-Ph-4-F | $CH_3$ | Smp. 114-116°C |

| Verb. Nr. | $R_1$ | $R_2$ | $R_3$ | Physikal. Daten |
|---|---|---|---|---|
| 1.60 | Cl | O-Ph-4-$CH_3$ | $CH_3$ | Smp. 165°C |
| 1.61 | Cl | O-Ph-3,4-$(CH_3)_2$ | $CH_3$ | Smp. 152°C |
| 1.62 | Cl | O-Ph-3-$CF_3$ | $CH_3$ | Smp. 109°C |
| 1.63 | Cl | O-Ph-3,5-$(CH_3)_2$ | $CH_3$ | Smp. 150°C |
| 1.64 | Cl | S-Ph | $CH_3$ | Smp. 138-140°C |
| 1.65 | Cl | S-Ph-4-$C(CH_3)_3$ | $CH_3$ | Smp. 128°C |
| 1.66 | Cl | O-Ph-4-$C_2H_5$ | $CH_3$ | Smp. 153°C |
| 1.67 | Cl | O-Ph-2,4-$(Cl)_2$ | $CH_3$ | Smp. 187-189°C |
| 1.68 | Cl | S-Ph-2,5-$(Cl)_2$ | $CH_3$ | Smp. 200-204°C |
| 1.69 | Cl | O-Ph-3-$OCF_3$ | $CH_3$ | Smp. 149-151°C |
| 1.70 | Cl | O-Ph-3,4-$(CH_3)_2$ | $CF_3$ | Smp. 95°C |
| 1.71 | Cl | S-Ph-4-F | $CH_3$ | Smp. 140-151°C |
| 1.72 | Cl | O-Ph-4-CN | $CH_3$ | Smp. 158-160°C |
| 1.73 | Cl | O-Ph-2,4-$(CH_3)_2$ | $CH_3$ | Smp. 188-190°C |
| 1.74 | Cl | O-Ph-4-Cl | $CF_3$ | Smp. 76-78°C |
| 1.75 | Cl | O-Ph-3,5-$(CH_3)_2$ | $CF_3$ | Smp. 112°C |
| 1.76 | Cl | O-Ph-4-$CH_3$ | $CF_3$ | Smp. 122°C |
| 1.77 | Cl | O-Ph-2,4-$(Cl)_2$ | $CF_3$ | Oel |
| 1.78 | Cl | S-Ph-4-$C(CH_3)_3$ | $CF_3$ | Smp. 119°C |
| 1.79 | Cl | S-Ph-4-$C(CH_3)_3$ | Ph | Smp. 131°C |
| 1.80 | Cl | O-Ph-2-F | $CF_3$ | Smp. 113°C |
| 1.81 | Cl | O-Ph-2,3-$(Cl)_2$ | $CF_3$ | Smp. 107°C |
| 1.82 | Cl | O-Ph-2-O-Ph | $CH_3$ | Smp. 109-115°C |
| 1.83 | H | O-Ph-4-$OC_2H_5$ | $CH_3$ | Smp. 50-65°C |
| 1.84 | H | O-Ph-2-Cl,4-$CF_3$ | $CH_3$ | Smp. 85-96°C |
| 1.85 | Cl | -O-Ph-3,4$(OCH_2O)$ | $CH_3$ | Smp. 145-149°C |
| 1.86 | Cl | -O-Ph-2-$OC_2H_5$ | $CH_3$ | Smp. 147-149°C |
| 1.87 | Cl | -O-Ph-4-$OCH_3$ | $CH_3$ | Smp. 151-153°C |
| 1.88 | Cl | -O-Ph-2-$OCH_3$ | $CH_3$ | Smp. 160-165°C |
| 1.89 | Cl | -O-Ph-4-$OC_3H_7$(n) | $CH_3$ | Smp. 176-179°C |
| 1.90 | Cl | -O-Ph-3-$OCH_3$ | $CH_3$ | Smp. 130-131°C |
| 1.91 | Cl | -O-Ph-3,5-$(OCH_3)_2$ | $CH_3$ | Smp. 136-139°C |
| 1.92 | Cl | -S-Ph-4-$OCH_3$ | $CH_3$ | Smp. 134-138°C |
| 1.93 | Cl | O-Ph | $CF_3$ | |
| 1.94 | Cl | O-Ph | Ph | |

| Verb. Nr. | $R_1$ | $R_2$ | $R_3$ | Physikal. Daten |
|---|---|---|---|---|
| 1.95 | Cl | O-Ph-2-Cl | $CF_3$ | |
| 1.96 | Cl | O-PH-2-F | $CF_3$ | |
| 1.97 | Cl | O-Ph-2-F | Ph | |
| 1.98 | Cl | O-Ph-4-Cl | $CF_3$ | |
| 1.99 | Cl | O-Ph-4-F | $CF_3$ | |
| 1.100 | Cl | O-Ph-4-CN | $CF_3$ | |
| 1.101 | Cl | O-Ph-4-OPh | $CF_3$ | |
| 1.102 | Cl | O-Ph-4-OPh | Ph | |
| 1.103 | Cl | O-Ph-4-Ph | $CF_3$ | |
| 1.104 | Cl | O-Ph-4-$C(CH_3)_3$ | $CH_3$ | |
| 1.105 | Cl | O-Ph-4-$C(CH_3)_3$ | $CF_3$ | |
| 1.106 | Cl | O-Ph-4-$C(CH_3)_3$ | Ph | |
| 1.107 | Cl | O-Ph-4-O-$CF_3$ | $CF_3$ | |
| 1.108 | Cl | S-Ph | $CF_3$ | |
| 1.109 | Cl | S-Ph-4-F | $CF_3$ | |
| 1.110 | Cl | O-Ph-3-OPh | $CF_3$ | |
| 1.111 | Cl | O-Ph-2,4-$(CH_3)_2$ | $CF_3$ | |
| 1.112 | Cl | O-Ph-2-(n)$C_3H_7$ | $CH_3$ | |
| 1.113 | Cl | O-Ph-4-(n)$C_3H_7$ | $CH_3$ | |
| 1.114 | Cl | O-Ph-4-$CH(CH_3)_3$ | $CH_3$ | |
| 1.115 | Cl | O-Ph-2-$CH(CH_3)_2$ | $CH_3$ | |
| 1.116 | Cl | O-Ph-3-$CH(CH_3)_2$ | $CH_3$ | |
| 1.117 | Cl | O-Ph-2-$CH(CH_3)CH_2CH_3$ | $CH_3$ | |
| 1.118 | Cl | O-Ph-4-$CH(CH_3)CH_2CH_3$ | $CH_3$ | |
| 1.119 | Cl | O-Ph-2-$C(CH_3)_3$ | $CH_3$ | |
| 1.120 | Cl | O-Ph-3-$C(CH_3)_3$ | $CH_3$ | |
| 1.123 | Cl | O-Ph-2-$OCH(CH_3)_2$ | $CH_3$ | |
| 1.124 | Cl | O-Ph-4-$OC_4H_9$(n) | $CH_3$ | |
| 1.125 | Cl | O-Ph-3-$N(C_2H_5)_2$ | $CH_3$ | |
| 1.126 | Cl | O-Ph-2,3-$(OCH_3)_2$ | $CH_3$ | |
| 1.127 | Cl | O-Ph-3-$N(CH_3)_2$ | $CH_3$ | |
| 1.129 | Cl | O-Ph-3,4,5-$(OCH_3)_3$ | $CH_3$ | |

| Verb. Nr. | $R_1$ | $R_2$ | $R_3$ | Physikal. Daten |
|---|---|---|---|---|
| 1.131* | 5-Cl | 6-(O-Ph-4-$OC_2H_5$) | $CH_3$ | Smp. 186°C |
| 1.132* | 6-Cl | 5-(O-Ph-4-$OC_2H_5$) | $CH_3$ | Smp. 161°C |

* Einzelisomere

Tabelle 2:

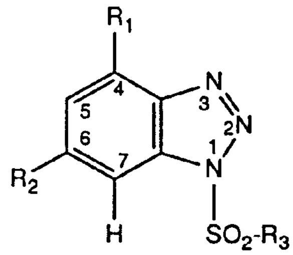

(Ph = Phenyl)

| Verb. Nr. | $R_1$ | $R_2$ | $R_3$ | Physikal. Daten |
|---|---|---|---|---|
| 2.1 | Cl | O-Ph | $CH_3$ | |
| 2.2 | Cl | O-Ph-3-Cl | $CH_3$ | |
| 2.3 | Cl | O-Ph-4-Cl | $CH_3$ | |
| 2.4 | Cl | O-Ph-4-Cl | $CH(CH_3)_2$ | |
| 2.5 | $CH_3$ | O-Ph-4-Cl | $CH_3$ | |
| 2.6 | $CH_3$ | O-Ph-2-F | $CH_3$ | |
| 2.7 | $CH_3$ | O-Ph-2,3-$(Cl)_2$ | $CH_3$ | |
| 2.8 | $CH_3$ | O-Ph-4-Cl | $CH_2$-Ph | |
| 2.9 | Cl | O-Ph-2-F | $CH_3$ | |
| 2.10 | Cl | O-Ph-2-Cl | $CH_3$ | |
| 2.11 | Cl | O-Ph-4-Cl | Ph | |
| 2.12 | Cl | O-Ph-4-Cl | $CH_2$-Ph | |
| 2.13 | Br | O-Ph-4-Cl | $CH_3$ | |
| 2.14 | $NO_2$ | O-Ph-4-Cl | $CH_3$ | |
| 2.15 | $CF_3$ | O-Ph-2-Cl | $CH_3$ | |
| 2.16 | $OCHF_2$ | O-Ph-4-Cl | $CH_3$ | |
| 2.17 | Cl | O-Ph-4-(O-Ph-4-F) | $CH_3$ | |
| 2.18 | Cl | O-Ph-4-Ph | $CH_3$ | |
| 2.19 | Br | O-Ph-4-Ph | $CH_3$ | |
| 2.20 | Br | O-Ph-4-(O-Ph-4-F) | $CH_3$ | |
| 2.21 | Br | O-Ph-3-O-Ph | $CH_3$ | |
| 2.22 | Cl | O-Ph-4-Cl | Ph-4-$CH_3$ | |
| 2.23 | Cl | O-Ph-4-$OC_2H_5$ | $CH_3$ | |
| 2.24 | Cl | O-Ph-4-$OCH_3$ | $CH_3$ | |
| 2.25 | Cl | O-Ph-3,5-$(OCH_3)_2$ | $CH_3$ | |
| 2.26 | $CH_3$ | O-Ph-4-O-Ph | $CH_3$ | |
| 2.27 | F | O-Ph-2-F | $CH_3$ | |

| Verb. Nr. | $R_1$ | $R_2$ | $R_3$ | Physikal. Daten |
|---|---|---|---|---|
| 2.28 | H | O-Ph-4-Cl | $CH_3$ | |
| 2.29 | H | S-Ph-2-Cl | $CH_3$ | |
| 2.30 | H | O-Ph-4-$OCH_3$ | $CH_3$ | |
| 2.31 | H | O-Ph-4-O-Ph | $CH_3$ | |
| 2.32 | H | O-Ph-2-Cl-4-F | $CH_3$ | |
| 2.33 | H | O-Ph-3-Cl | $C_6H_5$ | |
| 2.34 | H | O-Ph | $CH(CH_3)_3$ | |
| 2.35 | H | S-Ph | $CH_3$ | |
| 2.36 | H | O-Ph-4-O-Ph | $CH_3$ | |
| 2.37 | H | O-Ph-2,3-$(Cl)_2$ | $CH_3$ | |
| 2.38 | Cl | -O-Ph-4-$CH_3$ | $CH_3$ | |
| 2.39 | Cl | -O-Ph-4-$NO_2$ | $CH_3$ | |
| 2.40 | Cl | -S-Ph-4-Cl | $CH_3$ | |
| 2.41 | Cl | -S-Ph-4-$OCH_3$ | $CH_3$ | |
| 2.42 | Cl | -S-Ph | $CH_3$ | |
| 2.43 | Cl | -O-Ph-2,4-$Cl_2$ | $CH_3$ | |
| 2.44 | Cl | -O-Ph-2,3-$Cl_2$ | $CH_3$ | |
| 2.45 | Cl | -O-Ph-2,3-$(CH_3)_2$ | $CH_3$ | |
| 2.46 | Cl | -O-Ph-4-F | $CH_3$ | |
| 2.47 | Cl | -O-Ph-4-$CH_3$ | $CH_3$ | |
| 2.48 | Cl | -O-Ph-2-Cl,4-F | $CH_3$ | |
| 2.49 | Cl | -O-Ph-2,4-$(CH_3)_2$ | $CH_3$ | |
| 2.50 | Cl | -S-Ph-4-F | $CH_3$ | |

Tabelle 3:

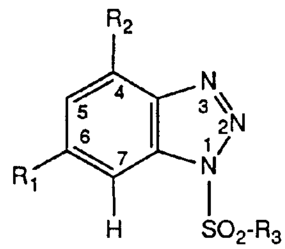

(Ph = Phenyl)

| Verb. Nr. | $R_1$ | $R_2$ | $R_3$ | Physikal. Daten |
|---|---|---|---|---|
| 3.1 | Cl | O-Ph | $CH_3$ | |
| 3.2 | Cl | O-Ph-3-Cl | $CH_3$ | |
| 3.3 | Cl | O-Ph-4-Cl | $CH_3$ | |
| 3.4 | Cl | O-Ph-4-Cl | $CH(CH_3)_2$ | |
| 3.5 | $CH_3$ | O-Ph-4-Cl | $CH_3$ | |
| 3.6 | $CH_3$ | O-Ph-2-F | $CH_3$ | |
| 3.7 | $CH_3$ | O-Ph-2,3-$(Cl)_2$ | $CH_3$ | |
| 3.8 | $CH_3$ | O-Ph-4-Cl | $CH_2$-Ph | |
| 3.9 | Cl | O-Ph-2-F | $CH_3$ | |
| 3.10 | Cl | O-Ph-2-Cl | $CH_3$ | |
| 3.11 | Cl | O-Ph-4-Cl | Ph | |
| 3.12 | Cl | O-Ph-4-Cl | $CH_2$-Ph | |
| 3.13 | Br | O-Ph-4-Cl | $CH_3$ | |
| 3.14 | $NO_2$ | O-Ph-4-Cl | $CH_3$ | |
| 3.15 | $CF_3$ | O-Ph-2-Cl | $CH_3$ | |
| 3.16 | $OCHF_2$ | O-Ph-4-Cl | $CH_3$ | |
| 3.17 | Cl | O-Ph-4-(O-Ph-4-F) | $CH_3$ | |
| 3.18 | Cl | O-Ph-4-Ph | $CH_3$ | |
| 3.19 | Br | O-Ph-4-Ph | $CH_3$ | |
| 3.20 | Br | O-Ph-4-(O-Ph-4-F) | $CH_3$ | |
| 3.21 | Br | O-Ph-3-O-Ph | $CH_3$ | |
| 3.22 | Cl | O-Ph-4-Cl | Ph-4-$CH_3$ | |
| 3.23 | Cl | O-Ph-4-$OC_2H_5$ | $CH_3$ | |
| 3.24 | Cl | O-Ph-4-$OCH_3$ | $CH_3$ | |
| 3.25 | Cl | O-Ph-3,5-$(OCH_3)_2$ | $CH_3$ | |
| 3.26 | $CH_3$ | O-Ph-4-O-Ph | $CH_3$ | |

| Verb. Nr. | $R_1$ | $R_2$ | $R_3$ | Physikal. Daten |
|---|---|---|---|---|
| 3.27 | F | O-Ph-2-F | $CH_3$ | |
| 3.28 | H | O-Ph-4-Cl | $CH_3$ | |
| 3.29 | H | S-Ph-2-F | $CH_3$ | |
| 3.30 | H | O-Ph-4-$OCH_3$ | $CH_3$ | |
| 3.31 | H | O-Ph-4-O-Ph | $CH_3$ | |
| 3.32 | H | O-Ph-2-Cl-4-F | $CH_3$ | |
| 3.33 | H | O-Ph-3-Cl | $C_6H_5$ | |
| 3.34 | H | O-Ph | $CH(CH_3)_3$ | |
| 3.35 | H | S-Ph | $CH_3$ | |
| 3.36 | H | O-Ph-4-Ph | $CH_3$ | |
| 3.37 | H | O-Ph-2,3-$(Cl)_2$ | $CH_3$ | |
| 3.38 | Cl | -O-Ph-4-$CH_3$ | $CH_3$ | |
| 3.39 | Cl | -O-Ph-4-$NO_2$ | $CH_3$ | |
| 3.40 | Cl | -S-Ph-4-Cl | $CH_3$ | |
| 3.41 | Cl | -S-Ph-4-$OCH_3$ | $CH_3$ | |
| 3.42 | Cl | -S-Ph | $CH_3$ | |
| 3.43 | Cl | -O-Ph-2,4-$Cl_2$ | $CH_3$ | |
| 3.44 | Cl | -O-Ph-2,3-$Cl_2$ | $CH_3$ | |
| 3.45 | Cl | -O-Ph-2,3-$(CH_3)_2$ | $CH_3$ | |
| 3.46 | Cl | -O-Ph-4-F | $CH_3$ | |
| 3.47 | Cl | -O-Ph-4-$CH_3$ | $CH_3$ | |
| 3.48 | Cl | -O-Ph-2-Cl,4-F | $CH_3$ | |
| 3.49 | Cl | -O-Ph-2,4-$(CH_3)_2$ | $CH_3$ | |
| 3.50 | Cl | -S-Ph-4-F | $CH_3$ | |

Tabelle 4:

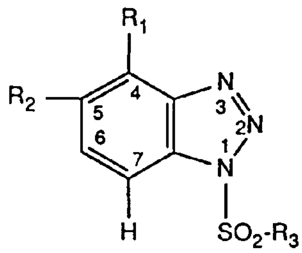

(Ph = Phenyl)

| Verb. Nr. | $R_1$ | $R_2$ | $R_3$ | Physikal. Daten |
|---|---|---|---|---|
| 4.1 | Cl | O-Ph | $CH_3$ | |
| 4.2 | Cl | O-Ph-3-Cl | $CH_3$ | |
| 4.3 | Cl | O-Ph-4-Cl | $CH_3$ | |
| 4.4 | Cl | O-Ph-4-Cl | $CH(CH_3)_2$ | |
| 4.5 | $CH_3$ | O-Ph-4-Cl | $CH_3$ | |
| 4.6 | $CH_3$ | O-Ph-2-F | $CH_3$ | |
| 4.7 | $CH_3$ | O-Ph-2,3-$(Cl)_2$ | $CH_3$ | |
| 4.8 | $CH_3$ | O-Ph-4-Cl | $CH_2$-Ph | |
| 4.9 | Cl | O-Ph-2-F | $CH_3$ | |
| 4.10 | Cl | O-Ph-2-Cl | $CH_3$ | |
| 4.11 | Cl | O-Ph-4-Cl | Ph | |
| 4.12 | Cl | O-Ph-4-Cl | $CH_2$-Ph | |
| 4.13 | Br | O-Ph-4-Cl | $CH_3$ | |
| 4.14 | $NO_2$ | O-Ph-4-Cl | $CH_3$ | |
| 4.15 | $CF_3$ | O-Ph-2-Cl | $CH_3$ | |
| 4.16 | $OCHF_2$ | O-Ph-4-Cl | $CH_3$ | |
| 4.17 | Cl | O-Ph-4-(O-Ph-4-F) | $CH_3$ | |
| 4.18 | Cl | O-Ph-4-Ph | $CH_3$ | |
| 4.19 | Br | O-Ph-4-Ph | $CH_3$ | |
| 4.20 | Br | O-Ph-4-(O-Ph-4-F) | $CH_3$ | |
| 4.21 | Br | O-Ph-3-O-Ph | $CH_3$ | |
| 4.22 | Cl | O-Ph-4-Cl | Ph-4-$CH_3$ | |
| 4.23 | Cl | O-Ph-4-$OC_2H_5$ | $CH_3$ | |
| 4.24 | Cl | O-Ph-4-$OCH_3$ | $CH_3$ | |
| 4.25 | Cl | O-Ph-3,5-$(OCH_3)_2$ | $CH_3$ | |
| 4.26 | $CH_3$ | O-Ph-4-(O-Ph) | $CH_3$ | |

| Verb. Nr. | $R_1$ | $R_2$ | $R_3$ | Physikal. Daten |
|---|---|---|---|---|
| 4.27 | F | O-Ph-2-F | $CH_3$ | |
| 4.28 | H | O-Ph-4-Cl | $CH_3$ | |
| 4.29 | H | S-Ph-2-F | $CH_3$ | |
| 4.30 | H | O-Ph-4-$OCH_3$ | $CH_3$ | |
| 4.31 | H | O-Ph-4-O-Ph | $CH_3$ | |
| 4.32 | H | O-Ph-2-Cl-4-F | $CH_3$ | |
| 4.33 | H | O-Ph-3-Cl | $C_6H_5$ | |
| 4.34 | H | O-Ph | $CH(CH_3)_3$ | |
| 4.35 | H | S-Ph | $CH_3$ | |
| 4.36 | H | O-Ph-4-O-Ph | $CH_3$ | |
| 4.37 | H | O-Ph-2,3-$(Cl)_2$ | $CH_3$ | |
| 4.38 | H | O-Ph-4-$OC_2H_5$ | $CH_3$ | Smp. 133-135°C |
| 4.39 | Cl | -O-Ph-4-$CH_3$ | $CH_3$ | |
| 4.40 | Cl | -O-Ph-4-$NO_2$ | $CH_3$ | |
| 4.41 | Cl | -S-Ph-4-Cl | $CH_3$ | |
| 4.42 | Cl | -S-Ph-4-$OCH_3$ | $CH_3$ | |
| 4.43 | Cl | -S-Ph | $CH_3$ | |
| 4.44 | Cl | -O-Ph-2,4-$Cl_2$ | $CH_3$ | |
| 4.45 | Cl | -O-Ph-2,3-$Cl_2$ | $CH_3$ | |
| 4.46 | Cl | -O-Ph-2,3-$(CH_3)_2$ | $CH_3$ | |
| 4.47 | Cl | -O-Ph-4-F | $CH_3$ | |
| 4.48 | Cl | -O-Ph-4-$CH_3$ | $CH_3$ | |
| 4.49 | Cl | -O-Ph-2-Cl,4-F | $CH_3$ | |
| 4.50 | Cl | -O-Ph-2,4-$(CH_3)_2$ | $CH_3$ | |
| 4.51 | Cl | -S-Ph-4-F | $CH_3$ | |

Tabelle 5:

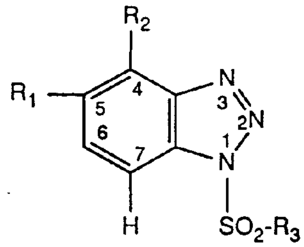

(Ph = Phenyl)

| Verb. Nr. | $R_1$ | $R_2$ | $R_3$ | Physikal. Daten |
|---|---|---|---|---|
| 5.1 | Cl | O-Ph | $CH_3$ | |
| 5.2 | Cl | O-Ph-3-Cl | $CH_3$ | |
| 5.3 | Cl | O-Ph-4-Cl | $CH_3$ | |
| 5.4 | Cl | O-Ph-4-Cl | $CH(CH_3)_2$ | |
| 5.5 | $CH_3$ | O-Ph-4-Cl | $CH_3$ | |
| 5.6 | $CH_3$ | O-Ph-2-F | $CH_3$ | |
| 5.7 | $CH_3$ | O-Ph-2,3-$(Cl)_2$ | $CH_3$ | |
| 5.8 | $CH_3$ | O-Ph-4-Cl | $CH_2$-Ph | |
| 5.9 | Cl | O-Ph-2-F | $CH_3$ | |
| 5.10 | Cl | O-Ph-2-Cl | $CH_3$ | |
| 5.11 | Cl | O-Ph-4-Cl | Ph | |
| 5.12 | Cl | O-Ph-4-Cl | $CH_2$-Ph | |
| 5.13 | Br | O-Ph-4-Cl | $CH_3$ | |
| 5.14 | $NO_2$ | O-Ph-4-Cl | $CH_3$ | |
| 5.15 | $CF_3$ | O-Ph-2-Cl | $CH_3$ | |
| 5.16 | $OCHF_2$ | O-Ph-4-Cl | $CH_3$ | |
| 5.17 | Cl | O-Ph-4-(O-Ph-4-F) | $CH_3$ | |
| 5.18 | Cl | O-Ph-4-Ph | $CH_3$ | |
| 5.19 | Br | O-Ph-4-Ph | $CH_3$ | |
| 5.20 | Br | O-Ph-4-(O-Ph-4-F) | $CH_3$ | |
| 5.21 | Br | O-Ph-3-O-Ph | $CH_3$ | |
| 5.22 | Cl | O-Ph-4-Cl | Ph-4-$CH_3$ | |
| 5.23 | Cl | O-Ph-4-$OC_2H_5$ | $CH_3$ | |
| 5.24 | Cl | O-Ph-4-$OCH_3$ | $CH_3$ | |
| 5.25 | Cl | O-Ph-3,5-$(OCH_3)_2$ | $CH_3$ | |
| 5.26 | $CH_3$ | O-Ph-4-O-Ph | $CH_3$ | |

| Verb. Nr. | $R_1$ | $R_2$ | $R_3$ | Physikal. Daten |
|---|---|---|---|---|
| 5.27 | F | O-Ph-2-F | $CH_3$ | |

Formulierungsbeispiele für Wirkstoffe der Formel I (% = Gewichtsprozent)

| 2.1. Spritzpulver | a) | b) | c) |
|---|---|---|---|
| Wirkstoff aus den Tabellen | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyethylenglykolether (7-8 Mol Ethylenoxid) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| 2.2. Emulsions-Konzentrat | |
|---|---|
| Wirkstoff aus den Tabellen | 10 % |
| Octylphenolpolyethylenglykolether (4-5 Mol Ethylenoxid) | 3 % |
| Ca-Dodecylbenzolsulfonat | 3 % |
| Ricinusölpolyglykolether (35 Mol Ethylenoxid) | 4 % |
| Cyclohexanon | 34 % |
| Xylolgemisch | 50 % |

Aus diesem Konzentrat können durch Verdünnen mit Wasser Emulsionen jeder gewünschten Konzentration hergestellt werden.

| 2.3. Stäubemittel | a) | b) |
|---|---|---|
| Wirkstoff aus den Tabellen | 5 % | 8 % |
| Talkum | 95 % | - |
| Kaolin | - | 92 % |

Man erhält anwendungsfertige Stäubemittel, indem der Wirkstoff mit dem Träger vermischt auf einer geeigneten Mühle vermahlen wird.

2.4. Extruder Granulat

| | |
|---|---|
| Wirkstoff aus den Tabellen | 10 % |
| N-Ligninsulfonat | 2 % |

| | |
|---|---|
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

2.5.Umhüllungs-Granulat

| | |
|---|---|
| Wirkstoff aus den Tabellen | 3 % |
| Polyethylenglykol (MG 200) | 3 % |
| Kaolin | 94 % |

(MG = Molekulargewicht)

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

2.6. Suspensions-Konzentrat

| | |
|---|---|
| Wirkstoff aus den Tabellen | 40 % |
| Ethylenglykol | 10 % |
| Nonylphenolpolyethylenglykolether (15 Mol Ethylenoxid) | 6 % |
| Na-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

3. Biologische Beispiele

Beispiel 3.1: Wirkung gegen Plasmopara viticola auf Reben

a) Residual-protektive Wirkung

Im 4-5 Blattstadium werden Rebensämlinge mit einer aus Spritzpulver des Wirkstoffes hergestellten Spritzbrühe (0,02 % Aktivsubstanz) besprüht. Nach 24 Stunden werden die behandelten Pflanzen mit einer Sporangiensuspension des Pilzes infiziert. Nach einer Inkubation während 6 Tagen bei 95-100 % relativer Luftfeuchtigkeit und 20°C wird der Pilzbefall beurteilt.

b) Residual-kurative Wirkung

Im 4-5 Blattstadium werden Rebensämlinge mit einer Sporangiensuspension des Pilzes infiziert. Nach einer Inkubation während 24 Stunden in einer Feuchtkammer bei 95- 100 % relativer Luftfeuchtigkeit und 20°C werden die infizierten Pflanzen getrocknet und mit einer aus Spritzpulver des Wirkstoffes hergestellten Spritzbrühe (0,02 % Aktivsubstanz) besprüht. Nach dem Antrocknen des Spritzbelages werden die behandelten Pflanzen wieder in die Feuchtkammer gebracht. Die Beurteilung des Pilzbefalls erfolgt 6 Tage nach der Infektion.

Verbindungen aus den Tabellen zeigen gegen Plasmopara viticola auf Reben eine sehr gute fungizide Wirkung, insbesondere die Wirkstoffe Nr. 1.6, 1.13, 1.14, 1.16, und 1.30 bewirken eine vollständige Unterdrückung des Pilzbefalls (Restbefall 0 bis 5 %). Unbehandelte aber infizierte Kontrollpflanzen weisen dagegen einen Plasmopara-Befall von 100 % auf.

Beispiel 3.2: Wirkung gegen Phytophthora auf Tomatenpflanzen

a) Residual-protektive Wirkung

Tomatenpflanzen werden nach 3-wöchiger Anzucht mit einer aus Spritzpulver des Wirkstoffes hergestellten Spritzbrühe (0,02 % Aktivsubstanz) besprüht. Nach 24 Stunden werden die behandelten Pflanzen mit einer Sporangiensuspension des Pilzes infiziert. Die Beurteilung des Pilzbefalls erfolgt nach einer Inkubation der infizierten Pflanzen während 5 Tagen bei 90-100 % relativer Luftfeuchtigkeit und 20°C.

b) Systemische Wirkung

Zu Tomatenpflanzen wird nach dreiwöchiger Anzucht eine aus Spritzpulver des Wirkstoffes hergestellte Spritzbrühe (0,002 % Aktivsubstanz bezogen auf das Erdvolumen) gegeben. Es wird dabei darauf geachtet, dass die Spritzbrühe nicht mit den oberirdischen Pflanzenteilen in Berührung kommt. Nach 48 Stunden werden die behandelten Pflanzen mit einer Sporangiensuspension des Pilzes infiziert. Die Beurteilung des Pilzbefalls erfolgt nach einer Inkubation der infizierten Pflanzen während 5 Tagen bei 90-100 % relativer Luftfeuchtigkeit und 20°C.

Verbindungen aus den Tabellen zeigen eine nachhaltige Wirkung (weniger als 20 % Pilzbefall). Mit den Verbindungen Nr. 1.3, 1.6, 1.13, 1.14, 1.16, 1.30 und 1.58 wird ein Befall praktisch vollständig verhindert (0 bis 5 % Befall). Unbehandelte aber infizierte Kontrollpflanzen weisen dagegen einen Phytophthora-Befall von 100 % auf.

Beispiel 3.3: Wirkung gegen Phytophthora auf Kartoffelpflanzen

a) Residual-protektive Wirkung

2-3 Wochen alte Kartoffelpflanzen (Sorte Bintje) werden nach 3-wöchiger Anzucht mit einer aus Spritzpulver des Wirkstoffes hergestellten Spritzbrühe (0,02 % Aktivsubstanz) besprüht. Nach 24 Stunden werden die behandelten Pflanzen mit einer Sporangiensuspension des Pilzes infiziert. Die Beurteilung des Pilzbefalls erfolgt nach einer Inkubation der infizierten Pflanzen während 5 Tagen bei 90-100 % relativer Luftfeuchtigkeit und 20°C.

b) Systemische Wirkung

Zu 2-3 Wochen alten Kartoffelpflanzen (Sorte Bintje) wird nach dreiwöchiger Anzucht eine aus Spritzpulver des Wirkstoffes hergestellte Spritzbrühe (0,002 % Aktivsubstanz bezogen auf das Erdvolumen) gegeben. Es wird dabei darauf geachtet, dass die Spritzbrühe nicht mit den oberirdischen Pflanzenteilen in Berührung kommt. Nach 48 Stunden werden die behandelten Pflanzen mit einer Sporangiensuspension des Pilzes infiziert. Die Beurteilung des Pilzbefalls erfolgt nach einer Inkubation der infizierten Pflanzen während 5 Tagen bei 90-100 % relativer Luftfeuchtigkeit und 20°C.

Verbindungen aus den Tabellen zeigen eine nachhaltige Wirkung (weniger als 20 % Pilzbefall). Mit den Verbindungen Nr. 1.6, 1.13, 1.14, 1.30 und 1.58 wird ein Befall praktisch vollständig verhindert (0 bis 5 % Befall). Unbehandelte aber infizierte Kontrollpflanzen weisen dagegen einen Phytophthora-Befall von 100 % auf.

**Patentansprüche**

1. Verbindungen der Formel I

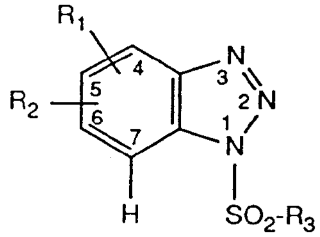

(I)

in welcher bedeuten:
$R_1$ Wasserstoff, Halogen, $C_1$-$C_4$-Alkyl, $C_1$-$C_4$-Alkoxy, $C_1$-$C_4$-Haloalkoxy mit mindestens zwei gleichen oder verschiedenen Halogenatomen, ferner $CF_3$, Nitro oder die Gruppe N(R')R", wobei R' und R" unabhängig voneinander $C_1$-$C_4$-Alkyl sind;
$R_2$ Phenoxy oder Phenylthio, welche unsubstituiert oder 1- bis 3-fach substituiert sind durch Halogen, $C_1$-$C_4$-Alkyl, $C_1$-$C_4$-Alkoxy, $C_1$-$C_4$-Haloalkoxy mit mindestens zwei gleichen oder verschiedenen Halogenatomen, ferner substituiert durch Cyano, Nitro, $C_2$-$C_4$-Alkenyl, $C_2$-$C_4$-Alkinyl oder in 3,4-Stellung durch einen Rest O-$CH_2$-O oder ferner substituiert durch Phenyl, 2-Phenylethinyl oder einen weiteren Phenoxyrest, der unsubstituiert oder durch Halogen, $C_1$-$C_4$-Alkyl und/oder $C_1$-$C_4$-Alkoxy substituiert ist;
$R_3$ Alkyl, Aryl oder Aralkyl mit maximal 14 C-Atomen, wobei diese Reste durch Halogen, $C_1$-$C_4$-Alkyl und/oder Nitro substituiert sind;
unter Einschluss der Stellungsisomeren von $R_1$ und $R_2$, sofern diese Substituenten die Positionen 5 oder 6 besetzen.

2. Verbindungen gemäss Anspruch 1 der Formel I in welcher bedeuten:
$R_1$ Wasserstoff, Halogen, $C_1$-$C_4$-Alkyl, Methoxy, Ethoxy, $CF_3$, $NO_2$, $C_1$-$C_2$-Haloalkoxy mit mindestens zwei Fluoratomen, oder ferner Diethylamin oder Dimethylamin;
und $R_2$ und $R_3$ die unter Formel I angegebenen Bedeutungen besitzen.

3. Verbindungen gemäss Anspruch 2 der Formel I in welcher bedeuten:
$R_1$ Wasserstoff, Fluor, Chlor, Brom, Methyl, Ethyl, Methoxy, $CF_3$, $NO_2$, $C_1$-$C_2$-Haloalkoxy mit mindestens 2 Fluoratomen, oder ferner Dimethylamin;
und $R_2$ und $R_3$ die unter Formel I angegebenen Bedeutungen besitzen.

4. Verbindungen gemäss Anspruch 3 der Formel I in welcher bedeuten:
$R_1$ Wasserstoff, Chlor, Brom, Methyl, Methoxy, $CF_3$, $NO_2$, Trifluormethoxy, Trifluorchlorethoxy, Difluormethoxy oder Dimethylamin;
und $R_2$ und $R_3$ die unter Formel I angegebenen Bedeutungen besitzen.

5. Verbindungen gemäss Anspruch 4 der Formel I in welcher bedeuten:
$R_1$ Chlor, Methyl, Methoxy, $CF_3$ oder Dimethylamin;
und $R_2$ und $R_3$ die unter Formel I angegebenen Bedeutungen besitzen.

6. Verbindungen gemäss Anspruch 1 der Formel I in welcher bedeuten:
$R_2$ Phenoxy oder Phenylthio, welche unsubstituiert oder 1- oder 2-fach substituiert sind durch Fluor, Chlor oder Brom, $C_1$-$C_4$-Alkyl, $C_1$-$C_4$-Alkoxy, $C_1$-$C_2$-Haloalkoxy mit mindestens 2 Fluoratomen, ferner substituiert durch Nitro, Cyano, Allyl, Propargyl, 2-Phenylethinyl oder einen weiteren Phenoxyrest, der unsubstituiert oder durch Fluor, Chlor, Brom oder Methyl substituiert ist;
und $R_1$ und $R_3$ die unter Formel I angegebenen Bedeutungen besitzen.

7. Verbindungen gemäss Anspruch 6 der Formel I in welcher bedeuten:
$R_2$ Phenoxy, das unsubstituiert oder substituiert ist durch Fluor, Chlor, Brom, $C_1$ -$C_4$-Alkyl, $C_1$-$C_4$-Alkoxy, $C_1$-$C_2$-Haloalkoxy mit mindestens 2 Fluoratomen, ferner substituiert durch Cyano, Nitro, Allyl, Propargyl, 2-Phenylethinyl oder einen weiteren Phenoxyrest, der unsubstituiert oder durch Fluor, Chlor, Brom oder Methyl substituiert ist;
und $R_1$ und $R_3$ die unter Formel I angegebenen Bedeutungen besitzen.

8. Verbindungen gemäss Anspruch 7 der Formel I in welcher bedeuten:
$R_2$ Phenylthio, das unsubstituiert oder substituiert ist durch Fluor, Chlor, $C_1$-$C_2$-Alkyl, $C_1$-$C_2$-Alkoxy, $C_1$-$C_2$-Haloalkoxy mit mindestens 2 Fluoratomen, ferner substituiert durch Nitro, Allyl, Propargyl oder 2-Phenylethinyl;
und $R_1$ und $R_3$ die unter Formel I angegebenen Bedeutungen besitzen.

9. Verbindungen gemäss Anspruch 8 der Formel I in welcher bedeuten:
$R_2$ Phenoxy, das unsubstituiert oder substituiert ist durch Chlor, Brom, Methyl, tert.Butyl, Methoxy, Ethoxy, Trifluormethoxy, Cyano, Nitro, Allyl, Propargyl, 2-Phenylethinyl oder einen weiteren Phenoxyrest, der unsubstituiert oder durch Fluor, Brom oder Methyl substituiert ist;
und $R_1$ und $R_3$ die unter Formel I angegebenen Bedeutungen besitzen.

**10.** Verbindungen gemäss Anspruch 1 der Formel I in welcher bedeuten:
$R_3$ $C_1$-$C_4$-Alkyl, durch Halogen substituiertes $C_1$-$C_4$-Alkyl, Phenyl, durch Halogen, $C_1$-$C_4$-Alkyl und/oder Nitro substituiertes Phenyl, Benzyl oder durch Halogen, $C_1$-$C_4$-Alkyl und/oder Nitro substituiertes Benzyl; und $R_1$ und $R_2$ die unter Formel I angegebenen Bedeutungen besitzen.

**11.** Verbindungen gemäss Anspruch 10 der Formel I in welcher bedeuten:
$R_3$ $C_1$-$C_4$-Alkyl, durch Fluor oder Chlor substituiertes Alkyl, Phenyl, durch Chlor, Methyl oder Nitro substituiertes Phenyl, Benzyl oder durch Chlor, Methyl oder Nitro substituiertes Benzyl;
und $R_1$ und $R_2$ die unter Formel I angegebenen Bedeutungen besitzen.

**12.** Verbindungen gemäss Anspruch 1 der Formel I in welcher bedeuten:
$R_1$ Wasserstoff, Halogen, $C_1$-$C_4$-Alkyl, Methoxy, Ethoxy, $CF_3$, $NO_2$, $C_1$-$C_2$-Haloalkoxy mit mindestens 2 Fluoratomen, oder ferner Diethylamin oder Dimethylamin;
$R_2$ Phenoxy oder Phenylthio, welche unsubstituiert oder 1- oder 2-fach substituiert sind durch Fluor, Chlor oder Brom, $C_1$-$C_4$-Alkyl, $C_1$-$C_4$-Alkoxy, $C_1$-$C_2$-Haloalkoxy mit mindestens 2 Fluoratomen, ferner substituiert durch Cyano, Nitro, Allyl, Propargyl, 2-Phenylethinyl oder einen weiteren Phenoxyrest, der unsubstituiert oder durch Fluor, Chlor, Brom oder Methyl substituiert ist;
$R_3$ $C_1$-$C_4$-Alkyl, durch Halogen substituiertes $C_1$-$C_4$-Alkyl, Phenyl, durch Halogen, $C_1$-$C_4$-Alkyl und/oder Nitro substituiertes Phenyl, Benzyl oder durch Halogen, $C_1$-$C_4$-Alkyl und/oder Nitro substituiertes Benzyl.

**13.** Verbindungen gemäss Anspruch 12 der Formel I in welcher bedeuten:
$R_1$ Wasserstoff, Fluor, Chlor, Brom, Methyl, Ethyl, Methoxy, $CF_3$, $NO_2$, $C_1$-$C_2$-Haloalkoxy mit mindestens 2 Fluoratomen, oder ferner Dimethylamin;
$R_2$ Phenoxy, das unsubstituiert oder substituiert ist durch Fluor, Chlor, Brom, $C_1$-$C_4$-Alkyl, $C_1$-$C_4$-Alkoxy, $C_1$-$C_2$-Haloalkoxy mit mindestens 2 Fluoratomen, ferner substituiert durch Cyano, Nitro, Allyl, Propargyl, 2-Phenylethinyl oder einen weiteren Phenoxyrest, der unsubstituiert oder durch Fluor, Brom oder Methyl substituiert ist;
$R_3$ $C_1$-$C_4$-Alkyl, durch Fluor oder Chlor substituiertes Alkyl, Phenyl, durch Chlor, Methyl oder Nitro substituiertes Phenyl, Benzyl oder durch Chlor, Methyl oder Nitro substituiertes Benzyl.

**14.** Verbindungen gemäss Anspruch 13 der Formel I in welcher bedeuten:
$R_1$ Wasserstoff, Chlor, Brom, Methyl, Methoxy, $CF_3$, $NO_2$, Trifluormethoxy, Trifluorchlorethoxy, Difluormethoxy oder Dimethylamin;
$R_2$ Phenylthio, das unsubstituiert oder substituiert ist durch Fluor, Chlor, $C_1$ -$C_2$-Alkyl, $C_1$-$C_2$-Alkoxy, $C_1$-$C_2$-Haloalkoxy mit mindestens 2 Fluor- und/oder Chloratomen, ferner substituiert durch Nitro, Allyl, Propargyl oder 2-Phenylethinyl;
$R_3$ $C_1$-$C_4$-Alkyl, durch Fluor oder Chlor substituiertes Alkyl, Phenyl, durch Chlor, Methyl oder Nitro substituiertes Phenyl, Benzyl oder durch Chlor, Methyl oder Nitro substituiertes Benzyl;
und $R_1$ und $R_2$ die unter Formel I angegebenen Bedeutungen besitzen.

**15.** Verbindungen gemäss Anspruch 14 der Formel I in welcher bedeuten:
$R_1$ Chlor, Methyl, Methoxy, $CF_3$ oder Dimethylamin;
$R_2$ Phenoxy, das unsubstituiert oder substituiert ist durch Chlor, Brom, Methyl, tert.Butyl, Methoxy, Ethoxy, Trifluormethoxy, Cyano, Nitro, Allyl, Propargyl, 2-Phenylethinyl oder einen weiteren Phenoxyrest, der unsubstituiert oder durch Fluor, Brom oder Methyl substituiert ist;
$R_3$ $C_1$-$C_4$-Alkyl, durch Fluor oder Chlor substituiertes Alkyl, Phenyl, durch Chlor, Methyl oder Nitro substituiertes Phenyl, Benzyl oder durch Chlor, Methyl oder Nitro substituiertes Benzyl.

**16.** Eine Verbindung der Formel I gemäss Anspruch 1 aus der Gruppe:
1-N-Methansulfonyl-5-chlor-6-phenoxy-benztriazol;
1-N-Methansulfonyl-5-chlor-6-(2-chlorphenoxy)-benztriazol;
1-N-Methansulfonyl-5-chlor-6-(4-chlorphenoxy)-benztriazol;
1-N-Methansulfonyl-5-chlor-6-(2,4-dibromphenoxy)-benztriazol;
1-N-Methansulfonyl-5-chlor-6-(2-fluorphenoxy)-benztriazol;
1-N-Methansulfonyl-5-chlor-6-(4-ethoxyphenoxy)-benztriazol;
3-N-Methansulfonyl-5-chlor-6-phenoxy-benztriazol;
3-N-Methansulfonyl-5-chlor-6-(2-chlorphenoxy)-benztriazol;
3-N-Methansulfonyl-5-chlor-6-(4-chlorphenoxy)-benztriazol;
3-N-Methansulfonyl-5-chlor-6-(2,4-dibromphenoxy)-benztriazol;

3-N-Methansulfonyl-5-chlor-6-(2-fluorphenoxy)-benztriazol; und
3-N-Methansulfonyl-5-chlor-6-(4-ethoxyphenoxy)-benztriazol;
sowie die Gemische der 1-N- und 3-N-substituierten Strukturisomeren.

**17.** Verfahren zur Herstellung von Verbindungen der Formel I in Anspruch 1 gekennzeichnet durch Umsetzung einer Verbindung der Formel II

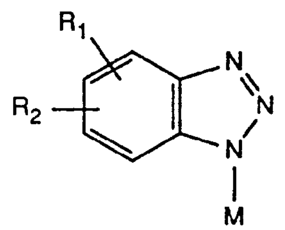

(II)

miit einer Verbindung der Formel III

$$Q\text{-}SO_2\text{-}R_3 \qquad \text{(III)}$$

worin $R_1$, $R_2$ und $R_3$ die unter Formel I angegebenen Bedeutungen besitzen, M Wasserstoff oder ein Alkalimetall, und Q ein Halogenatom oder den Rest $O\text{-}SO_2\text{-}R_3$ darstellen, in einem inerten Lösungsmittel, gegebenenfalls in Gegenwart einer Base, bei Temperaturen von -30° bis 180°C unter Normaldruck, vermindertem oder erhöhtem Druck; oder
durch Diazotierung einer Verbindung der Formel IV

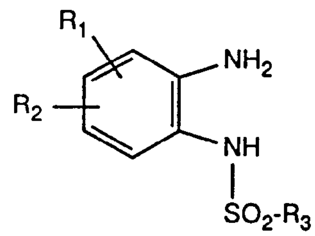

(IV)

worin $R_1$, $R_2$ und $R_3$ die unter Formel I angegebenen Bedeutungen besitzen, entweder
a) mit einem anorganischen Nitrit in einem Lösungsmittel in Gegenwart einer Säure bei Temperaturen von -30° bis 180°C; oder
b) mit einem organischen Nitrit in einem Lösungsmittel, bei Temperaturen von -30° bis 180°C unter Normaldruck oder erhöhtem Druck.

**18.** Mittel zur Bekämpfung oder Verhütung eines Befalls durch schädliche Mikroorganismen, dadurch gekennzeichnet, dass es als aktive Komponente mindestens eine Verbindung der Formel I gemäss Anspruch 1 enthält, zusammen mit einem geeigneten Trägermaterial.

**19.** Mittel gemäss Anspruch 18, dadurch gekennzeichnet, dass es als aktive Komponente mindestens eine Verbindung der Formel I gemäss einem der Ansprüche 2-11 enthält.

**20.** Mittel gemäss Anspruch 18, dadurch gekennzeichnet, dass es als aktive Komponente mindestens eine Verbindung der Formel I gemäss einem der Ansprüche 12-15 enthält.

**21.** Mittel gemäss Anspruch 18, dadurch gekennzeichnet, dass es als aktive Komponente mindestens eine Verbindung der Formel I gemäss Anspruch 16 enthält.

**22.** Verfahren zur Bekämpfung oder Verhütung eines Befalls von Kulturpflanzen durch phytopathogene Mikroorganismen, dadurch gekennzeichnet, dass man als Wirkstoff eine Verbindung der Formel I gemäss Anspruch 1 auf die Pflanze, auf Pflanzenteile oder ihren Standort appliziert.

**23.** Verfahren gemäss Anspruch 22, dadurch gekennzeichnet, dass man als Wirkstoff eine Verbindung gemäss einem der Ansprüche 2 bis 16 appliziert.

24. Verfahren gemäss Anspruch 22, dadurch gekennzeichnet, dass phytopathogene Fungi bekämpft werden.

25. Verfahren gemäss Anspruch 22, dadurch gekennzeichnet, dass es sich bei den phytopathogenen Fungi um Oomyceten handelt.

26. Verfahren gemäss Anspruch 25, dadurch gekennzeichnet, dass die Oomyceten die Species Plasmopara und Phytophthora sind.

27. Verwendung von Verbindungen gemäss Anspruch 1 zum Schutz von Pflanzen gegen den Befall durch phytopathogene Mikroorganismen.

28. Verwendung von Verbindungen gemäss Ansprüche 2 bis 16 zum Schutz von Pflanzen gegen den Befall durch phytopathogene Mikroorganismen.

29. Verfahren zur Herstellung eines agrochemischen Mittels in Anspruch 18, dadurch gekennzeichnet, dass man mindestens eine Verbindung der Formel I gemäss Anspruch 1 mit geeigneten festen oder flüssigen Zusatzstoffen und/oder Tensiden innig vermischt.

## Claims

1. A compound of formula I

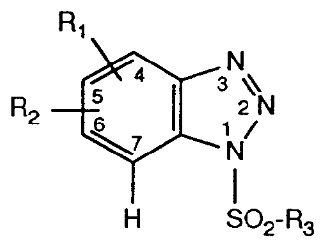

(I)

wherein:
$R_1$ is hydrogen, halogen, $C_1$-$C_4$alkyl, $C_1$-$C_4$alkoxy, $C_1$-$C_4$haloalkoxy having at least two identical or different halogen atoms, $CF_3$, nitro or the group N(R')R", wherein R' and R" are each independently of the other $C_1$-$C_4$alkyl;
$R_2$ is phenoxy or phenylthio each of which is unsubstituted or mono- to tri-substituted by halogen, $C_1$-$C_4$alkyl, $C_1$-$C_4$alkoxy, $C_1$-$C_4$haloalkoxy having at least two identical or different halogen atoms, or substituted by cyano, nitro, $C_2$-$C_4$alkenyl, $C_2$-$C_4$alkynyl or in the 3,4-position by an O-$CH_2$-O radical, or substituted by phenyl, 2-phenylethynyl or by a further phenoxy radical that is unsubstituted or substituted by halogen, $C_1$-$C_4$alkyl and/or by $C_1$-$C_4$alkoxy;
$R_3$ is alkyl, aryl or aralkyl having a maximum of 14 carbon atoms, these radicals being substituted by halogen, $C_1$-$C_4$alkyl and/or by nitro;
including the position isomers of $R_1$ and $R_2$, insofar as those substituents occupy positions 5 or 6.

2. A compound according to claim 1 of formula I wherein:
$R_1$ is hydrogen, halogen, $C_1$-$C_4$alkyl, methoxy, ethoxy, $CF_3$, $NO_2$, $C_1$-$C_2$haloalkoxy having at least two fluorine atoms, diethylamine or dimethylamine;
and $R_2$ and $R_3$ are as defined for formula I.

3. A compound according to claim 2 of formula I wherein:
$R_1$ is hydrogen, fluorine, chlorine, bromine, methyl, ethyl, methoxy, $CF_3$, $NO_2$, $C_1$-$C_2$-haloalkoxy having at least 2 fluorine atoms, or dimethylamine;
and $R_2$ and $R_3$ are as defined for formula I.

4. A compound according to claim 3 of formula I wherein:
$R_1$ is hydrogen, chlorine, bromine, methyl, methoxy, $CF_3$, $NO_2$, trifluoromethoxy, trifluorochloroethoxy, difluoromethoxy or dimethylamine;
and $R_2$ and $R_3$ are as defined for formula I.

5. A compound according to claim 4 of formula I wherein:
$R_1$ is chlorine, methyl, methoxy, $CF_3$ or dimethylamine;
and $R_2$ and $R_3$ are as defined for formula I.

6. A compound according to claim 1 of formula I wherein:
$R_2$ is phenoxy or phenylthio each of which is unsubstituted or mono- or di-substituted by fluorine, chlorine, bromine, $C_1$-$C_4$alkyl, $C_1$-$C_4$alkoxy, $C_1$-$C_2$haloalkoxy having at least 2 fluorine atoms, nitro, cyano, allyl, propargyl, 2-phenylethynyl or by a further phenoxy radical that is unsubstituted or substituted by fluorine, chlorine, bromine or by methyl;
and $R_1$ and $R_3$ are as defined for formula I.

7. A compound according to claim 6 of formula I wherein:
$R_2$ is phenoxy that is unsubstituted or substituted by fluorine, chlorine, bromine, $C_1$-$C_4$-alkyl, $C_1$-$C_4$alkoxy, $C_1$-$C_2$haloalkoxy having at least 2 fluorine atoms, cyano, nitro, allyl, propargyl, 2-phenylethynyl or by a further phenoxy radical that is unsubstituted or substituted by fluorine, chlorine, bromine or by methyl;
and $R_1$ and $R_3$ are as defined for formula I.

8. A compound according to claim 7 of formula I wherein:
$R_2$ is phenylthio that is unsubstituted or substituted by fluorine, chlorine, $C_1$-$C_2$alkyl, $C_1$-$C_2$alkoxy, $C_1$-$C_2$haloalkoxy having at least 2 fluorine atoms, nitro, allyl, propargyl or by 2-phenylethynyl;
and R1 and $R_3$ are as defined for formula I.

9. A compound according to claim 8 of formula I wherein:
$R_2$ is phenoxy that is unsubstituted or substituted by chlorine, bromine, methyl, tert-butyl, methoxy, ethoxy, trifluoromethoxy, cyano, nitro, allyl, propargyl, 2-phenylethynyl or by a further phenoxy radical that is unsubstituted or substituted by fluorine, bromine or by methyl;
and $R_1$ and $R_3$ are as defined for formula I.

10. A compound according to claim 1 of formula I wherein:
$R_3$ is $C_1$-$C_4$alkyl; $C_1$-$C_4$alkyl substituted by halogen; phenyl; phenyl substituted by halogen, $C_1$-$C_4$alkyl and/or by nitro; benzyl; or benzyl substituted by halogen, $C_1$-$C_4$alkyl and/or by nitro;
and $R_1$ and $R_2$ are as defined for formula I.

11. A compound according to claim 10 of formula I wherein:
$R_3$ is $C_1$-$C_4$alkyl; alkyl substituted by fluorine or by chlorine; phenyl; phenyl substituted by chlorine, methyl or by nitro; benzyl; or benzyl substituted by chlorine, methyl or by nitro;
and $R_1$ and $R_2$ are as defined for formula I.

12. A compound according to claim 1 of formula I wherein:
$R_1$ is hydrogen, halogen, $C_1$-$C_4$alkyl, methoxy, ethoxy, $CF_3$, $NO_2$, $C_1$-$C_2$haloalkoxy having at least 2 fluorine atoms, diethylamine or dimethylamine;
$R_2$ is phenoxy or phenylthio each of which is unsubstituted or mono- or di-substituted by fluorine, chlorine, bromine, $C_1$-$C_4$alkyl, $C_1$-$C_4$alkoxy, $C_1$-$C_2$haloalkoxy having at least 2 fluorine atoms, cyano, nitro, allyl, propargyl, 2-phenylethynyl or by a further phenoxy radical that is unsubstituted or substituted by fluorine, chlorine, bromine or by methyl; and
$R_3$ is $C_1$-$C_4$alkyl; $C_1$-$C_4$alkyl substituted by halogen; phenyl; phenyl substituted by halogen, $C_1$-$C_4$alkyl and/or by nitro; benzyl; or benzyl substituted by halogen, $C_1$-$C_4$alkyl and/or by nitro.

13. A compound according to claim 12 of formula I wherein:
$R_1$ is hydrogen, fluorine, chlorine, bromine, methyl, ethyl, methoxy, $CF_3$, $NO_2$, $C_1$-$C_2$-haloalkoxy having at least 2 fluorine atoms, or dimethylamine;
$R_2$ is phenoxy that is unsubstituted or substituted by fluorine, chlorine, bromine, $C_1$-$C_4$-alkyl, $C_1$-$C_4$alkoxy, $C_1$-$C_2$haloalkoxy having at least 2 fluorine atoms, cyano, nitro, allyl, propargyl, 2-phenylethynyl or by a further phenoxy radical that is unsubstituted or substituted by fluorine, bromine or by methyl; and
$R_3$ is $C_1$-$C_4$alkyl; alkyl substituted by fluorine or by chlorine; phenyl; phenyl substituted by chlorine, methyl or by nitro; benzyl; or benzyl substituted by chlorine, methyl or by nitro.

14. A compound according to claim 13 of formula I wherein:
$R_1$ is hydrogen, chlorine, bromine, methyl, methoxy, $CF_3$, $NO_2$, trifluoromethoxy, trifluorochloroethoxy,

difluoromethoxy or dimethylamine;
$R_2$ is phenylthio that is unsubstituted or substituted by fluorine, chlorine, $C_1$-$C_2$alkyl, $C_1$-$C_2$alkoxy, $C_1$-$C_2$haloalkoxy having at least 2 fluorine and/or chlorine atoms, nitro, allyl, propargyl or by 2-phenylethynyl; and
$R_3$ is $C_1$-$C_4$alkyl; alkyl substituted by fluorine or by chlorine; phenyl; phenyl substituted by chlorine, methyl or by nitro; benzyl; or benzyl substituted by chlorine, methyl or by nitro,
and $R_1$ and $R_2$ are as defined for formula I.

**15.** A compound according to claim 14 of formula I wherein:
$R_1$ is chlorine, methyl, methoxy, $CF_3$ or dimethylamine;
$R_2$ is phenoxy that is unsubstituted or substituted by chlorine, bromine, methyl, tert-butyl, methoxy, ethoxy, trifluoromethoxy, cyano, nitro, allyl, propargyl, 2-phenylethynyl or by a further phenoxy radical that is unsubstituted or substituted by fluorine, bromine or by methyl; and
$R_3$ is $C_1$-$C_4$alkyl; alkyl substituted by fluorine or by chlorine; phenyl; phenyl substituted by chlorine, methyl or by nitro; benzyl; or benzyl substituted by chlorine, methyl or by nitro.

**16.** A compound of formula I according to claim 1 from the group:
1-N-methanesulfonyl-5-chloro-6-phenoxy-benzotriazole;
1-N-methanesulfonyl-5-chloro-6-(2-chlorophenoxy)-benzotriazole;
1-N-methanesulfonyl-5-chloro-6-(4-chlorophenoxy)-benzotriazole;
1-N-methanesulfonyl-5-chloro-6-(2,4-dibromophenoxy)-benzotriazole;
1-N-methanesulfonyl-5-chloro-6-(2-fluorophenoxy)-benzotriazole;
1-N-methanesulfonyl-5-chloro-6-(4-ethoxyphenoxy)-benzotriazole;
3-N-methanesulfonyl-5-chloro-6-phenoxy-benzotriazole;
3-N-methanesulfonyl-5-chloro-6-(2-chlorophenoxy)-benzotriazole;
3-N-methanesulfonyl-5-chloro-6-(4-chlorophenoxy)-benzotriazole;
3-N-methanesulfonyl-5-chloro-6-(2,4-dibromophenoxy)-benzotriazole;
3-N-methanesulfonyl-5-chloro-6-(2-fluorophenoxy)-benzotriazole; and
3-N-methanesulfonyl-5-chloro-6-(4-ethoxyphenoxy)-benzotriazole;
or a mixture of the 1-N- and 3-N-substituted structural isomers.

**17.** A process for the preparation of a compound of formula I according to claim 1, which comprises reacting a compound of formula II

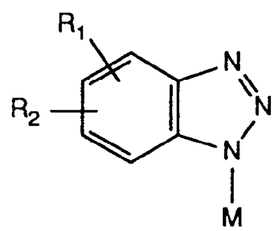

(II)

with a compound of formula III

$$Q\text{-}SO_2\text{-}R_3 \qquad \text{(III)},$$

wherein $R_1$, $R_2$ and $R_3$ are as defined for formula I and M is hydrogen or an alkali metal and Q is a halogen atom or the radical $O$-$SO_2$-$R_3$, in an inert solvent, in the presence or absence of a base, at temperatures from -30° to 180°C, under normal, reduced or elevated pressure; or
diazotizing a compound of formula IV

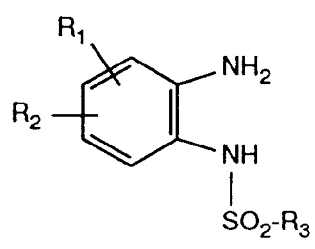

(IV),

wherein $R_1$, $R_2$ and $R_3$ are as defined for formula I, either

a) with an inorganic nitrite in a solvent, in the presence of an acid, at temperatures from -30° to 180°C; or

b) with an organic nitrite in a solvent, at temperatures from -30° to 180°C, under normal pressure or elevated pressure.

**18.** A composition for controlling or preventing an attack by harmful microorganisms, which comprises as active ingredient at least one compound of formula I according to claim 1, together with a suitable carrier.

**19.** A composition according to claim 18, which comprises as active ingredient at least one compound of formula I according to any one of claims 2-11.

**20.** A composition according to claim 18, which comprises as active ingredient at least one compound of formula I according to any one of claims 12-15.

**21.** A composition according to claim 18, which comprises as active ingredient at least one compound of formula I according to claim 16.

**22.** A method of controlling or preventing an attack on cultivated plants by phyto-pathogenic microorganisms, which comprises applying as active ingredient to the plants, parts of plants or the locus thereof a compound of formula I according to claim 1.

**23.** A method according to claim 22, which comprises applying as active ingredient a compound according to any one of claims 2 to 16.

**24.** A method according to claim 22, wherein phytopathogenic fungi are controlled.

**25.** A method according to claim 22, wherein the phytopathogenic fungi are Oomycetes.

**26.** A method according to claim 25, wherein the Oomycetes are the species Plasmopara and Phytophthora.

**27.** The use of compounds according to claim 1 for protecting plants against attack by phytopathogenic microorganisms.

**28.** The use of compounds according to claims 2 to 16 for protecting plants against attack by phytopathogenic microorganisms.

**29.** A process for the preparation of an agrochemical composition according to claim 18, which comprises homogeneously mixing at least one compound of formula I according to claim 1 with suitable solid or liquid adjuvants and/or surfactants.

## Revendications

**1.** Composés de formule I

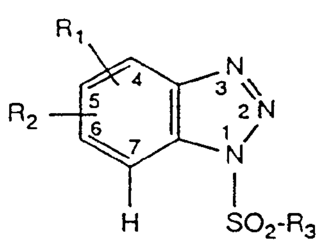

(I)

dans laquelle

$R_1$ représente l'hydrogène, un halogène, un groupe alkyle en C 1-C 4, alcoxy en C 1-C 4, halogénoalcoxy en C 1-C 4 contenant au moins 2 atomes d'halogènes identiques ou différents, ou encore $CF_3$, nitro, ou le groupe N(R')R" dans lequel R' et R" représentent chacun, indépendamment l'un de l'autre, un groupe alkyle en C 1-C 4;

$R_2$ représente un groupe phénoxy ou phénylthio non substitué ou portant 1 à 3 substituants halogéno, alkyle en C 1-C 4, alcoxy en C 1-C 4, halogénoalcoxy en C 1-C 4 contenant au moins 2 atomes d'halogènes identiques ou différents, ou encore substitué par des groupes cyano, nitro, alcényle en C 2-C 4, alcynyle en C 2-C 4, ou par un groupe $O-CH_2-O$ en position 3,4, ou encore substitué par des groupes phényle, 2-phényléthynyle ou par un autre groupe phénoxy lui-même non substitué ou substitué par des halogènes, des groupes alkyle en C 1-C 4 et/ou alcoxy en C 1-C 4;

$R_3$ représente un groupe alkyle, aryle ou aralkyle contenant au maximum 14 atomes de carbone, ces groupes pouvant être substitués par des halogènes, des groupes alkyle en C 1-C 4 et/ou nitro,

y compris les isomères de position de $R_1$ et $R_2$, lorsque ces substituants occupent les positions 5 ou 6.

**2.** Composés selon revendication 1, de formule I dans laquelle $R_1$ représente l'hydrogène, un halogène, un groupe alkyle en C 1-C 4, méthoxy, éthoxy, $CF_3$, $NO_2$, halogénoalcoxy en C 1-C 2 contenant au moins 2 atomes de fluor, ou encore diéthylamino ou diméthylamino;
et $R_2$ et $R_3$ ont les significations indiquées en référence à la formule I.

**3.** Composés selon revendication 2, de formule I dans laquelle $R_1$ représente l'hydrogène, le fluor, le chlore, le brome, un groupe méthyle, éthyle, méthoxy, $CF_3$, $NO_2$, halogénoalcoxy en C 1-C 2 contenant au moins 2 atomes de fluor, ou encore diméthylamino;
et $R_2$ et $R_3$ ont les significations indiquées en référence à la formule I.

**4.** Composés selon revendication 3, de formule I dans laquelle :
$R_1$ représente l'hydrogène, le chlore, le brome, un groupe méthyle, méthoxy, $CF_3$, $NO_2$, trifluorométhoxy, trifluorochloréthoxy, difluorométhoxy ou diméthylamino;
et $R_2$ et $R_3$ ont les significations indiquées en référence à la formule I.

**5.** Composés selon revendication 4, de formule I dans laquelle :
$R_1$ représente le chlore, un groupe méthyle, méthoxy, $CF_3$ ou diméthylamino;
et $R_2$ et $R_3$ ont les significations indiquées en référence à la formule I.

**6.** Composés selon revendication 1, de formule I dans laquelle :
$R_2$ représente un groupe phénoxy ou phénylthio non substitué ou portant 1 ou 2 substituants fluoro, chloro ou bromo, alkyle en C 1-C 4, alcoxy en C 1-C 4, halogénoalcoxy en C 1-C 2 contenant au moins 2 atomes de fluor, ou encore substitué par des groupes nitro, cyano, allyle, propargyle, 2-phényléthynyle ou par un autre groupe phénoxy lui-même non substitué ou substitué par le fluor, le chlore, le brome ou des groupes méthyle;
et $R_1$ et $R_3$ ont les significations indiquées en référence à la formule I.

**7.** Composés selon revendication 6, de formule I dans laquelle :
$R_2$ représente un groupe phénoxy non substitué ou substitué par le fluor, le chlore, le brome, des groupes alkyle en C 1-C 4, alcoxy en C 1-C 4, halogénoalcoxy en C 1-C 2 contenant au moins 2 atomes de fluor, ou encore substitué par des groupes cyano, nitro, allyle, propargyle, 2-phényléthynyle ou par un autre groupe phénoxy lui-même non substitué ou substitué par le fluor, le chlore, le brome ou un groupe méthyle;
et $R_1$ et $R_3$ ont les significations indiquées en référence à la formule I.

**8.** Composés selon revendication 7, de formule I dans laquelle :
$R_2$ représente un groupe phénylthio non substitué ou substitué par le fluor, le chlore, des groupes alkyle en C 1-C 2, alcoxy en C 1-C 2, halogénoalcoxy en C 1-C 2 contenant au moins 2 atomes de fluor, ou encore substitué par des groupes nitro, allyle, propargyle ou 2-phényléthynyle;
et $R_1$ et $R_3$ ont les significations indiquées en référence à la formule I.

**9.** Composés selon revendication 8, de formule I dans laquelle :
$R_2$ représente un groupe phénoxy non substitué ou substitué par le chlore, le brome, un groupe méthyle, tert-butyle, méthoxy, éthoxy, trifluorométhoxy, cyano, nitro, allyle, propargyle, 2-phényléthynyle ou par un autre groupe phénoxy lui-même substitué-par le fluor, le brome ou des groupes méthyle;
et $R_1$ et $R_3$ ont les significations indiquées en référence à la formule I.

**10.** Composés selon revendication 1, de formule I dans laquelle :

$R_3$ représente un groupe alkyle en C 1-C 4, un groupe alkyle en C 1-C 4 substitué par des halogènes, un groupe phényle, un groupe phényle substitué par des halogènes, des groupes alkyle en C 1-C 4 et/ou nitro, un groupe benzyle ou un groupe benzyle substitué par des halogènes, des groupes alkyle en C 1-C 4 et/ou nitro;
et $R_1$ et $R_2$ ont les significations indiquées en référence à la formule I.

**11.** Composés selon revendication 10, de formule I dans laquelle :
$R_3$ représente un groupe alkyle en C 1-C 4, un groupe alkyle substitué par le fluor ou le chlore, un groupe phényle, un groupe phényle substitué par le chlore, des groupes méthyle ou nitro, un groupe benzyle ou un groupe benzyle substitué par le chlore, des groupes méthyle ou nitro;
et $R_1$ et $R_2$ ont les significations indiquées en référence à la formule I.

**12.** Composés selon revendication 1, de formule I dans laquelle :
$R_1$ représente l'hydrogène, un halogène, un groupe alkyle en C 1-C 4, méthoxy, éthoxy, $CF_3$, $NO_2$, halogénoalcoxy en C 1-C 2 contenant au moins 2 atomes de fluor, ou encore diéthylamino ou diméthylamino;
$R_2$ représente un groupe phénoxy ou phénylthio non substitué ou portant 1 ou 2 substituants fluoro, chloro ou bromo, alkyle en C 1-C 4, alcoxy en C 1-C 4, halogénoalcoxy en C 1-C 2 contenant au moins 2 atomes de fluor, ou encore substitué par des groupes cyano, nitro, allyle, propargyle, 2-phényléthynyle ou par un autre groupe phénoxy lui-même substitué par le fluor, le chlore, le brome ou des groupes méthyle;
$R_3$ représente un groupe alkyle en C 1-C 4, un groupe alkyle en C 1-C 4 substitué par des halogènes, un groupe phényle, phényle substitué par des halogènes, des groupes alkyle en C 1-C 4 et/ou nitro, un groupe benzyle ou benzyle substitué par des halogènes, des groupes alkyle en C 1-C 4 et/ou nitro.

**13.** Composés selon revendication 12, de formule I dans laquelle :
$R_1$ représente l'hydrogène, le fluor, le chlore, le brome, des groupes méthyle, éthyle, méthoxy, $CF_3$, $NO_2$, halogénoalcoxy en C 1-C 2 contenant au moins 2 atomes de fluor, ou encore diméthylamino;
$R_2$ représente un groupe phénoxy non substitué ou substitué par le fluor, le chlore, le brome, des groupes alkyle en C 1-C 4, alcoxy en C 1-C 4, halogénoalcoxy en C 1-C 2 contenant au moins 2 atomes de fluor, ou encore substitué par des groupes cyano, nitro, allyle, propargyle, 2-phényléthynyle ou par un autre groupe phénoxy lui-même non substitué ou substitué par le fluor, le brome ou des groupes méthyle;
$R_3$ représente un groupe alkyle en C 1-C 4, alkyle substitué par le fluor ou le chlore, phényle, phényle substitué par le chlore, des groupes méthyle ou nitro, benzyle ou benzyle substitué par le chlore, des groupes méthyle ou nitro.

**14.** Composés selon revendication 13, de formule I dans laquelle :
$R_1$ représente l'hydrogène, le chlore, le brome, des groupes méthyle, méthoxy, $CF_3$, $NO_2$, trifluorométhoxy, trifluorochloréthoxy, difluorométhoxy ou diméthylamino;
$R_2$ représente un groupe phénylthio non substitué ou substitué par le fluor, le chlore, des groupes alkyle en C 1-C 2, alcoxy en C 1-C 2, halogénoalcoxy en C 1-C 2 contenant au moins 2 atomes de fluor et/ou de chlore, ou encore substitué par des groupes nitro, allyle, propargyle ou 2-phényléthynyle;
$R_3$ représente un groupe alkyle en C 1-C 4, alkyle substitué par le fluor ou le chlore, phényle, phényle substitué par le chlore, des groupes méthyle ou nitro, benzyle ou benzyle substitué par le chlore, des groupes méthyle ou nitro;
et $R_1$ et $R_2$ ont les significations indiquées en référence à la formule I.

**15.** Composés selon revendication 14, de formule I dans laquelle :
$R_1$ représente le chlore, des groupes méthyle, méthoxy, $CF_3$ ou diméthylamino;
$R_2$ représente un groupe phénoxy non substitué ou substitué par le chlore, le brome, des groupes méthyle, tert-butyle, méthoxy, éthoxy, trifluorométhoxy, cyano, nitro, allyle, propargyle, 2-phényléthynyle ou par un autre groupe phénoxy lui-même non substitué ou substitué par le fluor, le brome ou des groupes méthyle;
$R_3$ représente un groupe alkyle en C 1-C 4, alkyle substitué par le fluor ou le chlore, phényle, phényle substitué par le chlore, des groupes méthyle ou nitro, benzyle ou benzyle substitué par le chlore, des groupes méthyle ou nitro.

**16.** Un composé de formule I de la revendication 1, pris dans le groupe suivant :
1-N-méthane-sulfonyl-5-chloro-6-phénoxy-benzotriazole,
1-N-méthane-sulfonyl-5-chloro-6-(2-chlorophénoxy)-benzotriazole,
1-N-méthane-sulfonyl-5-chloro-6-(4-chlorophénoxy)-benzotriazole,

1-N-méthane-sulfonyl-5-chloro-6-(2,4-dibromophénoxybenzotriazole,
1-N-méthane-sulfonyl-5-chloro-6-(2-fluorophénoxy)-benzotriazole,
1-N-méthane-sulfçnyl-5-chloro-6-(4-éthoxyphénoxy)-benzotriazole,
3-N-méthane-sulfonyl-5-chloro-6-phénoxy-benzotriazole,
3-N-méthane-sulfonyl-5-chloro-6-(2-chlorophénoxy)-benzotriazole,
3-N-méthane-sulfonyl-5-chloro-6-(4-chlorophénoxy)-benzotriazole,
3-N-méthane-sulfonyl-5-chloro-6-(2,4-dibromophénoxy)-benzotriazole,
3-N-méthane-sulfonyl-5-chloro-6-(2-fluorophénoxy)-benzotriazole, et
3-N-méthane-sulfonyl-5-chloro-6-(4-éthoxyphénoxy)-benzotriazole;
ainsi que les mélanges des isomères de structure à substituants 1-N- et 3-N-.

**17.** Procédé de préparation des composés de formule I de la revendication 1, caractérisé en ce que : on fait réagir un composé de formule II

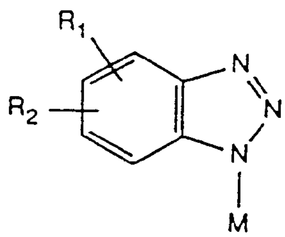

(II)

avec un composé de formule III

$$Q\text{-}SO_2\,R_3 \qquad \text{(III)}$$

$R_1$, $R_2$ et $R_3$ ayant dans ces formules les significations indiquées en référence à la formule I, M représentant l'hydrogène ou un métal alcalin et Q un atome d'halogène ou le groupe O-$SO_2$-$R_3$, dans un solvant inerte, éventuellement en présence d'une base, à des températures de -30 à 180°C, à pression normale, sous vide ou sous pression;
ou bien
on diazote un composé de formule IV

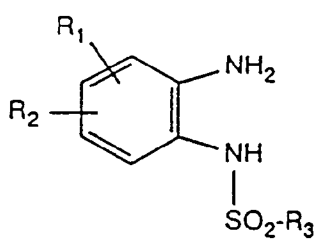

(IV)

dans laquelle $R_1$, $R_2$ et $R_3$ ont les significations indiquées en référence à la formule I, soit

a) par un nitrite minéral dans un solvant en présence d'un acide, à des températures de -30 à 180°C; soit

b) par un nitrite organique dans un solvant, à des températures de -30 à 180°C, à pression normale ou sous pression.

**18.** Produit pour combattre ou prévenir une attaque par des microorganismes nuisibles, caractérisé en ce qu'il contient au moins un composant actif consistant en un composé de formule I de la revendication 1, avec un véhicule approprié.

**19.** Produit selon revendication 18, caractérisé en ce qu'il contient au moins un composant actif consistant en un composé de formule I selon l'une des revendications 2 à 11.

**20.** Produit selon revendication 18, caractérisé en ce qu'il contient au moins un composant actif consistant en un composé de formule I selon l'une des revendications 12 à 15.

**21.** Produit selon revendication 18, caractérisé en ce qu'il contient au moins un composant actif consistant en un composé de formule I selon revendication 16.

**22.** Procédé pour combattre ou prévenir une attaque de.végétaux cultivés par des microorganismes phytopathogènes, caractérisé en ce que l'on applique sur la plante, des parties de la plante ou son habitat, une substance active consistant en un composé de formule I de la revendication 1.

**23.** Procédé selon revendication 22, caractérisé en ce que la substance active utilisée est un composé selon l'une des revendications 2 à 16.

**24.** Procédé selon revendication 22, caractérisé en ce que l'on combat des mycètes phytopathogènes.

**25.** Procédé selon revendication 22, caractérisé en ce que les mycètes phytopathogènes sont des oomycètes.

**26.** Procédé selon revendication 25, caractérisé en ce que les oomycètes appartiennent aux espèces Plasmopara et Phytophthora.

**27.** Utilisation des composés de formule I pour la protection des végétaux contre l'attaque par des micro-organismes phytopathogènes.

**28.** Utilisation des composés selon revendications 2 à 16 pour la protection des végétaux contre l'attaque par des microorganismes phytopathogènes.

**29.** Procédé de préparation d'un produit agro-chimique selon revendication 18, caractérisé en ce que l'on mélange intimement au moins un composé de formule I de la revendication 1 avec des additifs solides ou liquides et/ou des agents tensioactifs appropriés.